(19) Europäisches Patentamt
European Patent Office
Office européen des brevets

(11) **EP 4 617 255 A1**

(12) **EUROPEAN PATENT APPLICATION**
published in accordance with Art. 153(4) EPC


(43) Date of publication:
**17.09.2025 Bulletin 2025/38**

(21) Application number: **24865468.3**

(22) Date of filing: **11.09.2024**

(51) International Patent Classification (IPC):
***C07C 31/27*** (2006.01) ***C07B 61/00*** (2006.01)
***C07C 29/141*** (2006.01) ***C07C 45/50*** (2006.01)
***C07C 47/347*** (2006.01) ***C08F 20/10*** (2006.01)
***C08G 18/32*** (2006.01) ***C08G 59/24*** (2006.01)
***C08G 63/123*** (2006.01) ***C08G 64/02*** (2006.01)
***C09D 11/30*** (2014.01) ***C09D 201/02*** (2006.01)

(52) Cooperative Patent Classification (CPC):
**C07B 61/00; C07C 29/141; C07C 31/27; C07C 45/50; C07C 47/347; C08F 20/10; C08G 18/32; C08G 59/24; C08G 63/123; C08G 64/02; C09D 11/30; C09D 201/02**

(86) International application number:
**PCT/JP2024/032495**

(87) International publication number:
**WO 2025/057968 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.09.2023 JP 2023148245**
**07.02.2024 JP 2024017280**



(71) Applicant: **Mitsubishi Chemical Corporation**
**Tokyo 100-8251 (JP)**

(72) Inventors:
- **TASHIMA, Naoto**
  **Tokyo 100-8251 (JP)**
- **SUNAMI, Kazuaki**
  **Tokyo 100-8251 (JP)**
- **HINOISHI, Hiroki**
  **Tokyo 100-8251 (JP)**

(74) Representative: **Hoffmann Eitle**
**Patent- und Rechtsanwälte PartmbB**
**Arabellastraße 30**
**81925 München (DE)**


(54) **TRICYCLODECANEDIMETHANOL COMPOSITION, UV-CURABLE COMPOSITION, POLYMER COMPOSITION, AND TRICYCLODECANEDIMETHANOL COMPOSITION PRODUCTION METHOD**


(57) A tricyclodecanedimethanol composition comprising a chiral compound A, one of enantiomers of which is represented by the following formula (I), a chiral compound B, one of enantiomers of which is represented by the following formula (II), a chiral compound C, one of enantiomers of which is represented by the following formula (III), and a chiral compound D, one of enantiomers of which is represented by the following formula (IV), wherein the number of moles of the chiral compound A, Xa and the number of moles of the chiral compound B, Xb as measured by nuclear magnetic resonance spectrometry and a total number of moles of the chiral compound A, the chiral compound B, the chiral compound C, and the chiral compound D, Xt satisfy $Xa/Xt \leq 0.430$ and $Xb/Xt \geq 0.016$:

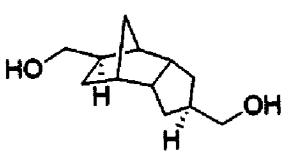
(I)

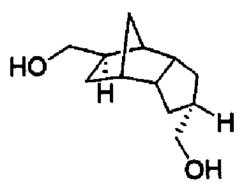
(II)


EP 4 617 255 A1

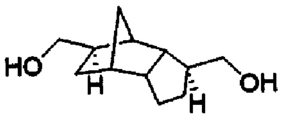 (III) 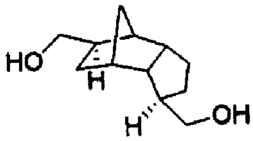 (IV)

**Description**

TECHNICAL FIELD

The present invention relates to a tricyclodecanedimethanol composition, an ultraviolet-curable composition, a polymer composition, and a method for producing a tricyclodecanedimethanol composition.

BACKGROUND ART

Tricyclodecanedimethanol (hereinafter abbreviated as "TCDDM") is a dihydric alcohol having an alicyclic structure. TCDDM is produced by subjecting dicyclopentadiene to hydroformylation reaction with carbon monoxide and hydrogen in the presence of a catalyst to obtain an aldehyde and then reducing the aldehyde with hydrogen (Patent Literature 1, Patent Literature 2, Patent Literature 3).

In the fields of molding materials, electronic materials, and display device members, materials having alicyclic molecular structures are used from the viewpoint of transparency, heat resistance, and low water absorbability.

As such materials having alicyclic molecular structures, polymers containing above-described TCDDM as their constituent are recently known to develop excellent performance in terms of hardness, transparency, heat resistance, and low water absorbability due to the alicyclic structure of TCDDM. TCDDM is attracting attention as a raw material for synthesizing molding materials such as polyester and polycarbonate.

TCDDM derivatives synthesized using TCDDM as a raw material, such as diacrylic acid ester derivatives, dimethacrylic acid ester derivatives, and urethane acrylates are used as ultraviolet-curable compositions. Cured products obtained using such ultraviolet-curable compositions develop excellent performance in terms of surface hardness, heat resistance (glass transition temperature), thermal decomposition resistance, and development resistance due to the alicyclic structure of TCDDM, and are therefore attracting attention as electronic materials such as hard coats, antifouling coats, and resists and display device members.

TCDDM may industrially be produced by subjecting dicyclopentadiene to a hydroformylation reaction, then subjecting the resultant to a hydrogen reduction reaction, and then subjecting the resultant to distillation purification to obtain TCDDM.

In the hydroformylation reaction, the reactivity of the double bond of the norbornane ring of dicyclopentadiene is higher than that of the double bond of the five-membered ring. Therefore, after the first formyl group is attached to the norbornane ring, the second formyl group is attached to the double bond of the five-membered ring (Non-Patent Literature 1). As a result, it is known that TCDDM obtained after the hydrogen reduction reaction is a mixture of TCDDM (hereinafter referred to as "TCDDM composition") mainly composed of two or more types of isomers different in the binding position of a hydroxymethyl group in the five-membered ring (Non-Patent Literature 2).

CITATION LIST

PATENT LITERATURES

Patent Literature 1: Japanese Patent Laid-Open No. 2020-132624
Patent Literature 2: Japanese Patent Laid-Open No. 2021-520401
Patent Literature 3: International Publication No.

WO2023/277347

NON-PATENT LITERATURES

Non-Patent Literature 1: Hitachi Chemical technical report No. 51 (2008-7), pp. 7 to 12
Non-Patent Literature 2: Applied Catalyst, Vol. 19, pp. 259 to 273 (1985).

SUMMARY OF THE INVENTION

## OBJECT OF THE INVENTION

The present inventors have newly found that even when a TCDDM composition obtained through distillation purification after hydrogen reduction reaction has fluidity just after distillation, handleability of the TCDDM composition may be reduced due to a reduction in fluidity during storage, that is, the TCDDM composition may have poor storage stability depending on the composition of the TCDDM composition.

If the fluidity of a TCDDM composition is reduced, there is such a problem that it is difficult to feed the TCDDM composition or a raw material mixture containing the TCDDM composition (hereinafter referred to as "TCDDM composition or the like") with a gear pump or the like or the TCDDM composition or the like is altered by heating performed to improve the fluidity thereof.

However, in Patent Literatures 1 to 3, there is no description about the fact that a TCDDM composition may have poor storage stability depending on the composition thereof and a method for improving the storage stability of a TCDDM composition.

It is therefore an object of the present invention to provide a TCDDM composition having excellent storage stability.

## SOLUTION TO PROBLEM

The present inventors discovered that appropriately controlling isomer ratios is effective in a mixture of TCDDM mainly composed of four types of isomers different in the binding position of the hydroxymethyl group. More specifically, they had knowledge that a TCDDM composition having excellent storage stability could be obtained by making composed ratio of the component a predetermined value or more because one component of four types of isomers has an action of improving the fluidity of a TCDDM composition and by making composed ratio of the component a predetermined value or less because another component of those has an action of reducing the fluidity of a TCDDM composition.

The main points of the present invention are as follows.

<1> A tricyclodecanedimethanol composition comprising a chiral compound A, one of enantiomers of which is represented by the following formula (I), a chiral compound B, one of enantiomers of which is represented by the following formula (II), a chiral compound C, one of enantiomers of which is represented by the following formula (III), and a chiral compound D, one of enantiomers of which is represented by the following formula (IV), wherein
the number of moles of the chiral compound A, Xa and the number of moles of the chiral compound B, Xb as measured by nuclear magnetic resonance spectrometry and a total number of moles of the chiral compound A, the chiral compound B, the chiral compound C, and the chiral compound D, Xt satisfy $Xa/Xt \leq 0.430$ and $Xb/Xt \geq 0.016$:

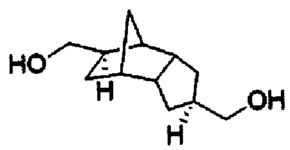 (I) 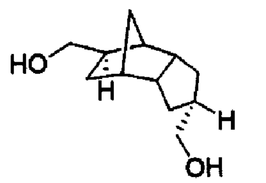 (II)

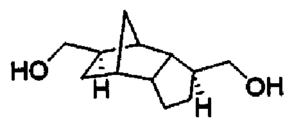 (III) 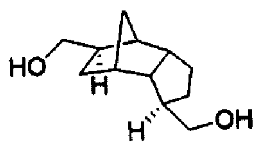 (IV)

<2> The tricyclodecanedimethanol composition according to <1>, wherein the Xb and the Xt satisfy $Xb/Xt \geq 0.020$.
<3> The tricyclodecanedimethanol composition according to <1> or <2>, wherein the Xa and the Xt satisfy $Xa/Xt \leq 0.400$.
<4> The tricyclodecanedimethanol composition according any one of <1> to <3>, wherein the Xb and the Xt satisfy $Xb/Xt \geq 0.027$.
<5> The tricyclodecanedimethanol composition according to any one of <1> to <4>, wherein the Xa and the Xt satisfy $Xa/Xt < 0.350$.
<6> The tricyclodecanedimethanol composition according to any one of <1> to <5>, wherein the number of moles of the chiral compound C, Xc as detected by NMR spectrometry and the Xt satisfy $Xc/Xt \geq 0.300$.
<7> The tricyclodecanedimethanol composition according to any one of <1> to <6>, wherein the number of moles of

the chiral compound D, Xd as detected by NMR spectrometry and the Xt satisfy $Xd/Xt \geq 0.240$.
<8> The tricyclodecanedimethanol composition according to any one of <1> to <7>, wherein the Xa, the Xb, and the number of moles of the chiral compound C, Xc and the number of moles of the chiral compound D, Xd as measured by nuclear magnetic resonance spectrometry satisfy $Xb/(Xa + Xc + Xd) \geq 0.010$.
<9> The tricyclodecanedimethanol composition according to any one of <1> to <8>, wherein the chiral compound B is detected at a retention time of 13.65 to 13.85 minutes when the composition is measured by gas chromatography (GC) under the following measurement conditions:

(Measurement Conditions)
Column: Capillary column (length 30 m × inner diameter 0.25 mm × film thickness 1 μm)
Liquid phase: 100% Dimethylpolysiloxane
Carrier gas: Helium
Carrier gas column flow rate: 1 mL/min
Split ratio: 1/30
Injection volume: 0.3 μL
Oven temperature: 160°C (no holding time) → heating-up at 5°C/min → 300°C (2-min holding time)
Inlet temperature: 200°C
Detector: Flame ionization detector (temperature 300°C)

<10> The tricyclodecanedimethanol composition according to <9>, wherein the chiral compound A is detected at a retention time of 13.85 to 14.05 minutes when the composition is measured by gas chromatography (GC) under the measurement conditions.
<11> An ultraviolet-curable composition derived from the tricyclodecanedimethanol composition according to any one of <1> to <10>.
<12> The ultraviolet-curable composition according to <11>, which is used for any one of a hard coating material, an antifouling coating material, a resist material, an ink-jet ink, and a material for 3D printers.
<13> A polymer composition derived from the tricyclodecanedimethanol composition according to any one of <1> to <10> or the ultraviolet-curable composition according to <11> or <12>.
<14> The polymer composition according to <13>, which comprises, as a polymer, at least one selected from the group consisting of a polyester-based resin, an epoxy-based resin, an acrylate-based resin, a polycarbonate-based resin, and a polyurethane-based resin.
<15> A method for producing a tricyclodecanedimethanol composition, the method comprising the steps of: subjecting dicyclopentadiene to hydroformylation to obtain tricyclodecanedicarbaldehyde; subjecting the tricyclodecanedicarbaldehyde to a reduction reaction to obtain a crude reaction liquid containing tricyclodecanedimethanol; and subjecting the crude reaction liquid to distillation purification to obtain the tricyclodecanedimethanol composition according to any one of <1> to <10>.
<16> The method for producing a tricyclodecanedimethanol composition according to <15>, wherein

a reaction pressure of the hydroformylation is 0.5 MPaG or more and 4.5 MPaG or less,
a temperature of the reduction reaction is 125°C or more and 350°C or less, and
distillation conditions of the distillation purification satisfy the following formula (1):

$$(Ya - Za) \times T/S \times 100 \leq 1.65 \quad (1)$$

(wherein
Ya: Mass ratio (unit: dimensionless number) of chiral compound A in crude reaction liquid supplied to distillation purification step
S: Total weight (unit: g) of chiral compound A, chiral compound B, chiral compound C, and chiral compound D in crude reaction liquid supplied to distillation purification step
Za: Mass ratio (unit: dimensionless number) of chiral compound A in distillate distilled off outside purification system from distillation tower in distillation purification step
T: Total weight (unit: g) of chiral compound A, chiral compound B, chiral compound C, and chiral compound D in distillate distilled off outside purification system from distillation tower in distillation purification step).

ADVANTAGEOUS EFFECTS OF THE INVENTION

The present invention makes it possible to provide a TCDDM composition having excellent storage stability. More specifically, the present invention makes it possible to provide a TCDDM composition having excellent storage stability by

appropriately selecting specific isomer ratios in such a manner that the fluidity of the TCDDM composition is not reduced even during long-term storage.

BRIEF DESCRIPTION OF THE DRAWINGS

[Figure 1] Figure 1 is a gas chromatogram of a TCDDM composition obtained in Example 2.
[Figure 2] Figure 2(a) is a $^{13}C$-NMR spectrum of the TCDDM composition obtained in Example 2 and Figure 2(b) is a $^{13}C$-NMR spectrum of a TCDDM composition obtained in Comparative Example 1, wherein indexes "A", "B", "C", and "D" attached to NMR peaks denote that these NMR peaks are respectively a peak corresponding to a "chiral compound A", a peak corresponding to a "chiral compound B", a peak corresponding to a "chiral compound C", and a peak corresponding to a "chiral compound D".

DETAILED DESCRIPTION OF THE INVENTION

Hereinbelow, the present invention will be described in detail. The present invention is not limited to the following description and can be carried out with any modification within the scope of the gist thereof.
Unless otherwise specified, a numerical range expressed herein using "to" means a range including numerical values written before and after "to" as a lower limit and an upper limit. "A to B" means A or more and B or less.
Unless otherwise specified, "comprising A or B" herein means "including A", "including B", and "including A and B".
"% by mass" herein represents the content of a predetermined component relative to a total amount of 100% by mass. "% by mass " and "% by weight" have the same meaning.
"Optional" or "optionally" herein means that the subsequently described circumstance may or may not occur, and therefore the description includes instances where the circumstance occurs and instances where the circumstance does not occur.
The term "about" used herein means that the stated value can vary by plus/minus 20%. For example, a temperature of about 75°C relative to 0 degrees Celsius refers to a range of 60°C to 90°C.
All steps described herein can be performed in any appropriate order unless otherwise specified herein or clearly contradicted by context.
Embodiments of the present invention will be described in detail below. The following description of constituent elements is an example of an embodiment of the present invention, and the present invention is not limited to the contents thereof.

<Tricyclodecanedimethanol Composition>

A tricyclodecanedimethanol composition of the present invention (hereinafter also referred to as "TCDDM composition") is a composition containing tricyclodecanedimethanol (hereinafter also referred to as "TCDDM").
The TCDDM composition is a mixture containing a chiral compound A, one of enantiomers of which is represented by the following formula (I), a chiral compound B, one of enantiomers of which is represented by the following formula (II), a chiral compound C, one of enantiomers of which is represented by the following formula (III), and a chiral compound D, one of enantiomers of which is represented by the following formula (IV).
As shown by the following general formulas (I) to (IV), the chiral compound A, the chiral compound B, the chiral compound C, and the chiral compound D each have one hydroxymethyl group ($CH_2OH$ group) in the six-membered ring moiety of the norbornane ring and another hydroxymethyl group in the five-membered ring moiety.

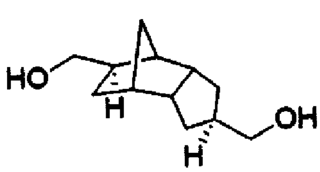 (I) 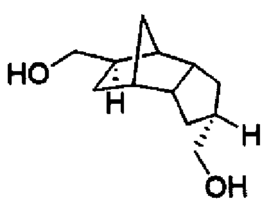 (II)

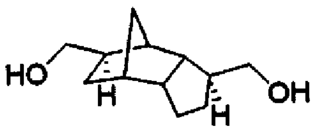 (III) 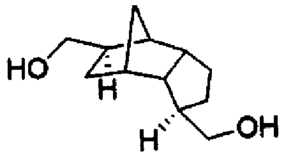 (IV)

As described above, each of the chiral compound A, the chiral compound B, the chiral compound C, and the chiral compound D in the present invention has R and S enantiomers (also referred to as optical isomers), but the difference between them does not matter and both of them are included.

Each of the chiral compound A, the chiral compound B, the chiral compound C, and the chiral compound D in the present invention has stereoisomers depending on whether the two hydroxymethyl groups are bound to the same side as or the opposite side to the bridgehead position of the norbornane ring. However, as shown by the formulas (I) to (IV), in each of the chiral compound A, the chiral compound B, the chiral compound C, and the chiral compound D in the present invention, the hydroxymethyl groups are bound to the same side as the bridgehead position of the norbornane ring. Further, the tricyclodecane skeleton has an endo form and an exo form, but the chiral compound A, the chiral compound B, the chiral compound C, and the chiral compound D in the present invention have only the endo form.

Hereinafter, "chiral compound A", "chiral compound B", "chiral compound C", and "chiral compound D" may simply be referred to as "compound A", "compound B", "compound C", and "compound D", respectively.

In the present invention, the number of moles of the chiral compound A, the number of moles of the chiral compound B, the number of moles of the chiral compound C, and the number of moles of the chiral compound D as measured by nuclear magnetic resonance spectrometry ($^{13}C$-NMR) are respectively defined as Xa, Xb, Xc, and Xd. The total number of moles of the chiral compound A, the chiral compound B, the chiral compound C, and the chiral compound D is defined as Xt (= Xa + Xb + Xc + Xd).

A method for measuring Xa, Xb, Xc, and Xd in the present invention will be described in detail in the section of Examples.

<Xa/Xt>

The upper limit of Xa/Xt of the TCDDM composition of the present invention calculated from the Xa and the Xt is $Xa/Xt \leq 0.430$ from the viewpoint of storage stability of the TCDDM composition. The Xa/Xt is preferably $Xa/Xt \leq 0.400$, more preferably $Xa/Xt \leq 0.370$, even more preferably $Xa/Xt \leq 0.360$, particularly preferably $Xa/Xt < 0.350$.

On the other hand, the lower limit of the Xa/Xt is not limited but may usually be $Xa/Xt \geq 0.250$ from the viewpoint that handleability of the TCDDM composition can appropriately be maintained, and is preferably $Xa/Xt \geq 0.270$, more preferably $Xa/Xt \geq 0.290$, even more preferably $Xa/Xt \geq 0.310$, particularly preferably $Xa/Xt \geq 0.330$.

The upper limit and the lower limit can freely be combined. For example, the Xa/Xt of the TCDDM composition of the present invention is not limited but may be $0.250 \leq Xa/Xt \leq 0.430$, and is preferably $0.270 \leq Xa/Xt \leq 0.400$, more preferably $0.290 \leq Xa/Xt \leq 0.370$, even more preferably $0.310 \leq Xa/Xt \leq 0.360$, particularly preferably $0.330 \leq Xa/Xt < 0.350$.

A means for controlling the value of the Xa/Xt is not limited, and a person skilled in the art can control the value of the Xa/Xt by appropriately optimizing production conditions according to well-known techniques. For example, the value of the Xa/Xt can be controlled by optimizing reaction conditions of hydroformylation, distillation conditions at the time when the obtained TCDDM composition is subjected to distillation purification, and the like.

<Xb/Xt>

The lower limit of Xb/Xt of the TCDDM composition of the present invention calculated from the Xb and the Xt is $Xb/Xt \geq 0.016$ from the viewpoint of storage stability of the TCDDM composition. The Xb/Xt is preferably $Xb/Xt \geq 0.020$, more preferably $Xb/Xt \geq 0.027$, even more preferably $Xb/Xt \geq 0.030$, particularly preferably $Xb/Xt \geq 0.031$, most preferably $Xb/Xt \geq 0.032$.

On the other hand, the upper limit of the Xb/Xt is not limited but may be $Xb/Xt \leq 0.13$ from the viewpoint that handleability of the TCDDM composition can appropriately be maintained by appropriately maintaining fluidity of the TCDDM composition. The Xb/Xt is preferably $Xb/Xt \leq 0.10$, more preferably $Xb/Xt \leq 0.048$, even more preferably $Xb/Xt \leq 0.046$, particularly preferably $Xb/Xt \leq 0.044$, most preferably $Xb/Xt \leq 0.042$.

The upper limit and the lower limit can freely be combined. For example, the Xb/Xt of the TCDDM composition of the present invention is not limited but may be $0.016 \leq Xb/Xt \leq 0.13$, and is preferably $0.020 \leq Xb/Xt \leq 0.10$, more preferably $0.027 \leq Xb/Xt \leq 0.048$, even more preferably $0.030 \leq Xb/Xt \leq 0.046$, particularly preferably $0.031 \leq Xb/Xt \leq 0.044$, most preferably $0.032 \leq Xb/Xt \leq 0.042$.

A means for controlling the value of the Xb/Xt is not limited, and a person skilled in the art can control the value of the Xb/Xt by appropriately optimizing production conditions according to well-known techniques. For example, the value of

the Xb/Xt can be controlled by optimizing reaction conditions of hydroformylation, distillation conditions at the time when the obtained TCDDM composition is subjected to distillation purification, and the like.

<Xc/Xt>

The lower limit of Xc/Xt of the TCDDM composition of the present invention calculated from the Xc and the Xt may be Xc/Xt ≥ 0.300 from the viewpoint of storage stability of the TCDDM composition. The Xc/Xt is preferably Xc/Xt ≥ 0.310, more preferably Xc/Xt ≥ 0.320, even more preferably Xc/Xt ≥ 0.330.
On the other hand, the upper limit of the Xc/Xt is not limited but may usually be Xc/Xt ≤ 0.380 from the viewpoint that handleability of the TCDDM composition can appropriately be maintained. The Xc/Xt is preferably Xc/Xt ≤ 0.360, more preferably Xc/Xt ≤ 0.350, even more preferably Xc/Xt ≤ 0.340.
The upper limit and the lower limit can freely be combined. For example, the Xc/Xt of the TCDDM composition of the present invention is not limited but may be 0.300 ≤ Xc/Xt ≤ 0.380, and is preferably 0.310 ≤ Xc/Xt ≤ 0.360, more preferably 0.320 ≤ Xc/Xt ≤ 0.350, even more preferably 0.330 ≤ Xc/Xt ≤ 0.340.
A means for controlling the value of the Xc/Xt is not limited, and a person skilled in the art can control the value of the Xc/Xt by appropriately optimizing production conditions according to well-known techniques. For example, the value of the Xc/Xt can be controlled by optimizing reaction conditions of hydroformylation, distillation conditions at the time when the obtained TCDDM composition is subjected to distillation purification, and the like.

<Xd/Xt>

The lower limit of Xd/Xt of the TCDDM composition of the present invention calculated from the Xd and the Xt is not limited but may be Xd/Xt ≥ 0.240 from the viewpoint of storage stability of the TCDDM composition. The Xd/Xt is preferably Xd/Xt ≥ 0.250, more preferably Xd/Xt ≥ 0.260, even more preferably Xd/Xt ≥ 0.270, particularly preferably Xd/Xt ≥ 0.280.
On the other hand, the upper limit of the Xd/Xt is not limited but may be Xd/Xt ≤ 0.370 from the viewpoint that handleability of the TCDDM composition can appropriately be maintained. The Xd/Xt is preferably Xd/Xt ≤ 0.350, more preferably Xd/Xt ≤ 0.330, even more preferably Xd/Xt ≤ 0.320, particularly preferably Xd/Xt < 0.310.
The upper limit and the lower limit can freely be combined. For example, the Xd/Xt of the TCDDM composition of the present invention is not limited but may be 0.240 ≤ Xd/Xt ≤ 0.370, and is preferably 0.250 ≤ Xd/Xt ≤ 0.350, more preferably 0.260 ≤ Xd/Xt ≤ 0.330, even more preferably 0.270 ≤ Xd/Xt ≤ 0.320, particularly preferably 0.280 ≤ Xd/Xt ≤ 0.310.
A means for controlling the value of the Xd/Xt is not limited, and a person skilled in the art can control the value of the Xd/Xt by appropriately optimizing production conditions according to well-known techniques. For example, the value of the Xd/Xt can be controlled by optimizing reaction conditions of hydroformylation, distillation conditions at the time when the obtained TCDDM composition is subjected to distillation purification, and the like.

<Xb/ (Xa + Xc + Xd)>

The lower limit of Xb/(Xa + Xc + Xd) of the TCDDM composition of the present invention calculated from the Xa, the Xb, the Xc, the Xd, and the Xt is not limited, but from the viewpoint of storage stability of the TCDDM composition, the Xb/(Xa + Xc + Xd) may be 0.010 or more (Xb/(Xa + Xc + Xd) ≥ 0.010) and is preferably 0.023 or more, more preferably 0.027 or more, even more preferably 0.032 or more.
On the other hand, the upper limit of the Xb/(Xa + Xc + Xd) is not limited, but from the viewpoint that handleability of the TCDDM composition can appropriately be maintained, the Xb/(Xa + Xc + Xd) may be 0.050 or less (Xb/(Xa + Xc + Xd) ≤ 0.050) and is preferably 0.045 or less, more preferably 0.040 or less, even more preferably 0.036 or less.
The upper limit and the lower limit can freely be combined. For example, the Xb/(Xa + Xc + Xd) of the TCDDM composition of the present invention is not limited but is preferably 0.010 or more and 0.050 or less, more preferably 0.023 or more and 0.045 or less, even more preferably 0.027 or more and 0.040 or less, particularly preferably 0.032 or more and 0.036 or less.
A means for controlling the value of the Xb/(Xa + Xc + Xd) is not limited, and a person skilled in the art can control the value of the Xb/(Xa + Xc + Xd) by appropriately optimizing production conditions according to well-known techniques. For example, the value of the Xb/(Xa + Xc + Xd) can be controlled by optimizing reaction conditions of hydroformylation, distillation conditions at the time when the obtained TCDDM composition is subjected to distillation purification, and the like.
The chiral compound B in the TCDDM composition of the present invention is a compound detected at a retention time of 13.65 to 13.85 minutes when the composition is measured by gas chromatography (GC) under the following measurement conditions.
The chiral compound A in the TCDDM composition of the present invention is a compound detected at a retention

time of 13.85 to 14.05 minutes when the composition is measured by gas chromatography (GC) under the following measurement conditions.

(Measurement Conditions)

Column: Capillary column (length 30 m × inner diameter 0.25 mm × film thickness 1 μm)
Liquid phase: 100% Dimethylpolysiloxane
Carrier gas: Helium
Carrier gas column flow rate: 1 mL/min
Split ratio: 1/30
Injection volume: 0.3 μL
Oven temperature: 160°C (no holding time) → heating-up at 5°C/min → 300°C (2-min holding time)
Inlet temperature: 200°C
Detector: Flame ionization detector (temperature 300°C)

The purity of the TCDDM composition of the present invention is not limited as long as the value of the Xa/Xt, the value of the Xb/Xt, and the like are satisfied, and the TCDDM composition of the present invention may be one whose purity has been increased by purification or the like or one containing impurities other than TCDDM.
The lower limit of total content of the chiral compound A, the chiral compound B, the chiral compound C, and the chiral compound D in the TCDDM composition of the present invention is not limited but is preferably 80% by mass or more, more preferably 90% by mass or more, even more preferably 95% by mass or more relative to 100% by mass of the total mass of the composition from the viewpoint of handleability of the TCDDM composition. On the other hand, the upper limit of the total content is not limited but is preferably high as much as possible, and may be 100% by mass relative to the total mass of the composition.

<Method for Producing TCDDM Composition>

A method for producing the above-described TCDDM composition is not limited, but as an embodiment, a method for producing a TCDDM composition of the present invention can be used.
The method for producing a TCDDM composition of the present invention is a production method including the steps of: subjecting dicyclopentadiene to hydroformylation to obtain tricyclodecanedicarbaldehydes, subjecting the tricyclodecanedicarbaldehydes to a reduction reaction to obtain a crude reaction liquid containing tricyclodecanedimethanol, and subjecting the crude reaction liquid to distillation purification to obtain a tricyclodecanedimethanol composition.
In this case, isomers are generated in the step of introducing formyl groups through hydroformylation, and therefore a mixture of tricyclodecanedicarbaldehydes, which are respectively precursors of the chiral compound A, the chiral compound B, the chiral compound C, and the chiral compound D, is obtained. Then, the chiral compound A, the chiral compound B, the chiral compound C, and the chiral compound D are obtained through the step of hydrogenation.
A method for controlling the ratio among the chiral compound A, the chiral compound B, the chiral compound C, and the chiral compound D in the present invention is not limited. The ratio among the chiral compound A, the chiral compound B, the chiral compound C, and the chiral compound D may be controlled by adjusting reaction conditions of hydroformylation or by isomerization using a technique such as heating or by subjecting the produced TCDDM composition to distillation purification.

<Hydroformylation Reaction of Dicyclopentadiene>

The method of hydroformylation of dicyclopentadiene is not limited and may be an ordinary method.
For example, according to the method disclosed in Japanese Patent Laid-Open No. 2001-10999, tricyclodecanedicarbaldehyde can be produced by hydroformylating dicyclopentadiene using hydrogen and carbon monoxide in the presence of a catalyst containing a rhodium compound and an organic phosphorus compound in a hydroformylation reaction solvent containing a hydrocarbon compound as shown by the following reaction formula (V).

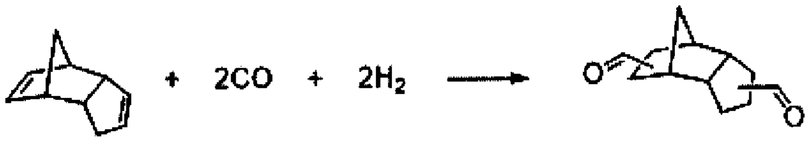

(V)

The rhodium compound used in this hydroformylation step is not limited by its precursor form as long as it forms a complex with an organic phosphorus compound and exhibits hydroformylation activity in the presence of hydrogen and carbon monoxide. Specifically, a catalytically-active rhodium metal hydride carbonyl phosphorus complex may be formed in a reaction vessel by introducing a catalyst precursor such as Rh (acac) $(CO)_2$, $Rh_2O_3$, $Rh_4$ $(CO)_{12}$, $Rh_6$ $(CO)_{16}$, or $Rh(NO_3)_3$ into a reaction mixture together with an organic phosphorus compound or a previously-prepared rhodium metal hydride carbonyl phosphorus complex catalyst may be introduced into a reaction vessel.

In a preferred specific example of the present invention, Rh (acac) $(CO)_2$ used as a rhodium precursor is reacted with an organic phosphorus compound in the presence of a solvent, and then the resultant is introduced into a reactor together with an excessive amount of a free organic phosphorus compound to form a catalytically-active rhodium-organic phosphorus complex catalyst.

The organic phosphorus compound that forms a catalyst for hydroformylation reaction with the rhodium compound may be a phosphite or a phosphine.

Among these, the phosphite is preferably a compound represented by the general formula, $P(-OR^1)(-OR^2)(-OR^3)$(wherein $R^1$, $R^2$, and $R^3$ are each an aryl group or an alkyl group that may be substituted) because such a compound is effective for the hydroformylation reaction of dicyclopentadiene.

Specific examples of $R^1$, $R^2$, and $R^3$ include: aryl groups, such as a phenyl group and a naphthyl group, which may be substituted with a methyl group, an ethyl group, an isopropyl group, an n-butyl group, a t-butyl group, a methoxy group, or the like; aliphatic alkyl groups such as a methyl group, an ethyl group, an isopropyl group, an n-butyl group, and a t-butyl group; alicyclic alkyl groups, such as a cyclopentyl group and a cyclohexyl group, which may be substituted with a lower alkyl group such as a methyl group, an ethyl group, an isopropyl group, an n-butyl group, or a t-butyl group.

Specific preferred examples of the phosphite include, but are not limited to, tris(2-t-butylphenyl)phosphite, tris(3-methyl-6-t-butylphenyl)phosphite, tris(3-methoxy-6-t-butylpheny)phosphite, tris(2,4-di-t-butylphenyl)phosphite, and di(2-t-butylphenyl) (t-butyl) phosphite. These phosphites may be used singly or in combination of two or more of them.

As the phosphine, an alkylphosphine having large steric hindrance is particularly effective for the hydroformylation reaction of dicyclopentadiene. Typical examples of such an alkylphosphine include, but are not limited to, tricyclopropylphosphine, tricyclobutylphosphine, tricyclopentylphosphine, tricyclohexylphosphine, tricycloheptylphosphine, and tricyclooctylphosphine. These phosphines may be used singly or in combination of two or more of them.

The amount of the organic phosphorus compound to be used is in the range of 1 to 400 molar times, preferably in the range of 3 to 200 molar times relative to the amount of rhodium metal in a hydroformylation reaction liquid. This makes it possible to obtain tricyclodecanedicarbaldehyde at a sufficient hydroformylation reaction rate.

The hydroformylation reaction of dicyclopentadiene can be performed without using a solvent, but can more appropriately be performed by using an organic solvent inactive against the reaction.

As will be described later, after the hydroformylation reaction is completed, layer separation is performed by bringing a reaction product liquid containing tricyclodecanedicarbaldehyde into contact with an alcohol so that the tricyclodecanedicarbaldehyde is extracted into an extraction solvent layer containing the alcohol while the catalyst components are left in a dihydroformylation reaction solvent layer. Therefore, the hydroformylation reaction solvent is preferably one that separates from an alcohol. Examples of such a solvent include an aromatic hydrocarbon compound, an aliphatic hydrocarbon compound, and an alicyclic hydrocarbon compound.

Examples of the aromatic hydrocarbon compound include methylbenzenes such as benzene, toluene, xylene, mesitylene, and pseudocumene, ethylbenzenes such as ethylbenzene, diethylbenzene, and triethylbenzene, and propylbenzenes such as isopropylbenzene, 1,3-diisopropylbenzene, and 1,4-diisopropylbenzene. Various alkylbenzenes other than these can also suitably be used.

Examples of the aliphatic hydrocarbon compound include pentane, hexane, heptane, octane, iso-octane, dodecane, and decane. The aliphatic hydrocarbon compound is not limited to these as long as it is liquid at standard temperature and pressure.

As the alicyclic hydrocarbon compound, cyclohexane, cyclooctane, cyclododecane, decalin, methylcyclohexane, or the like is suitably used.

These solvents may be used singly or in combination of two or more of them.

From the viewpoint of reaction efficiency, the solvent is used in such a manner that the concentration of dicyclopentadiene in the reaction liquid is preferably 10 to 95% by mass, particularly preferably about 30 to 90% by mass.

The amount of the rhodium catalyst to be used is usually 10 to 5000 ppm by weight, more preferably 50 to 2000 ppm by weight in terms of rhodium metal relative to the amount of dicyclopentadiene as a raw material. When rhodium is

used at 50 ppm or more, catalyst recovery is required.

The reaction pressure of hydroformylation reaction of dicyclopentadiene may be in the range of 0.5 MPaG or more to 4.5 MPaG or less from the viewpoint that the content of the chiral compound A (Xa/Xt) and the content of the chiral compound B (Xb/Xt) in a TCDDM composition to be obtained are controlled to fall within their respective desired ranges and the TCDDM composition of the present invention is efficiently produced. Specifically, from the viewpoint of appropriately maintaining the reaction rate of hydroformylation, the lower limit of reaction pressure of the hydroformylation reaction is preferably 0.5 MPaG or more, more preferably 1.0 MPaG or more, even more preferably 1.5 MPaG or more. On the other hand, from the viewpoint of controlling the value of Xa/Xt of a TCDDM composition to be obtained to be 0.430 or less, the upper limit of the reaction pressure is preferably 4.5 MPaG or less, more preferably 4.0 MPaG or less, even more preferably 3.5 MPaG or less.

The upper limit and the lower limit can freely be combined. For example, the reaction pressure of the hydroformylation reaction is preferably 0.5 MPaG or more and 4.5 MPaG or less, more preferably 1.0 MPaG or more and 4.0 MPaG or less, even more preferably 1.5 MPaG or more and 3.5 MPaG or less.

The reaction temperature of hydroformylation reaction of dicyclopentadiene is not limited and may usually be in the range of 40°C or more to 160°C or less. When the reaction temperature is lower, the reaction rate of hydroformylation tends to be lower. If the reaction temperature is too high, dicyclopentadiene and hydroformylation reaction products in the reaction liquid cause side reactions, and therefore the yield of tricyclodecanedicarbaldehyde tends to reduce. The reaction temperature may preferably be 80°C or more and 140°C or less.

The mole ratio between hydrogen and carbon monoxide in a hydrogen/carbon monoxide mixed gas used for the reaction can be selected from the range of 0.2 or more to 5.0 or less in terms of introduced gas composition (hydrogen/carbon monoxide). If the mole ratio between hydrogen and carbon monoxide in the hydrogen/carbon monoxide mixed gas falls outside this range, reaction activity of the hydroformylation reaction or aldehyde selectivity reduces.

As a reaction method for hydroformylation, a continuous feed method is used in which hydroformylation is performed while dicyclopentadiene as a raw material is fed alone or as a mixed solution with a solvent into a reactor containing a rhodium-organic phosphorus complex catalyst, a solvent, and a hydrogen/carbon monoxide mixed gas. The use of such a method makes it possible to reduce generation of cyclopentadiene, which inhibits the hydroformylation reaction, due to thermal decomposition of dicyclopentadiene in the reactor, thereby maintaining an appropriate reaction rate and a good yield. It is preferred that dicyclopentadiene is diluted with the above-described solvent to maintain its fluidity and is fed into the reactor at a temperature at which cyclopentadiene is not generated by depolymerization of dicyclopentadiene.

<Extraction of Tricyclodecanedicarbaldehyde>

After the hydroformylation reaction is completed, layer separation is performed by bringing a reaction product liquid into contact with an alcohol directly or after dilution with a hydrocarbon compound used in the reaction as a hydroformylation reaction solvent or another hydrocarbon compound so that tricyclodecanedicarbaldehyde as a product is extracted into the alcohol while the catalyst components are left in a hydroformylation reaction solvent layer.

Examples of the alcohol include a primary alcohol having 1 to 3 carbon atoms and a polyhydric alcohol having 2 to 6 carbon atoms.

Examples of the primary alcohol include methanol, ethanol, and propanol.

Examples of the polyhydric alcohol having 2 to 6 carbon atoms include ethylene glycol, 1,3-propanediol, 1,2-propanediol, 1,4-butanediol, 1,2-butanediol, 1,3-butanediol, 2,3-butanediol, and isomers of pentanediol, neopentyl glycol, hexanediol, glycerin, pentaerythritol, and trimethylolpropane.

Among these, methanol, ethylene glycol, propanediol, and butanediol are suitably used because they have relatively low boiling points, are inexpensive, and are easy to handle as a liquid. These extraction solvents may be used singly or in combination of two or more of them.

The extraction using an alcohol may be performed in the presence of water. Addition of water makes it easy to distribute the aldehyde and the catalyst components to different layers.

The reaction solvent used for the hydroformylation reaction and the extraction solvent are preferably different in density to achieve effective layer separation. A preferred example of a combination of the hydroformylation reaction solvent containing tricyclodecanedicarbaldehyde and the extraction solvent is a combination of methylcyclohexane as a reaction solvent and ethylene glycol as an extraction solvent or a combination of methylcyclohexane as a reaction solvent, methanol as an extraction solvent, and water.

The distribution of tricyclodecanedicarbaldehyde between the hydroformylation reaction solvent and the extraction solvent is equilibrium. On the other hand, rhodium and the organic phosphorus compound as catalyst components are substantially present only in the hydroformylation reaction solvent and are therefore present in the extraction solvent only at levels below their analytical limits.

The volume ratio between the extraction solvent used and the reaction product liquid is determined by the

solubility of tricyclodecanedicarbaldehyde in the extraction solvent and the amount of tricyclodecanedicarbaldehyde to be extracted. For example, when tricyclodecanedicarbaldehyde to be separated has high solubility in the extraction solvent and is present in the reaction product liquid at a low concentration, practical extraction of tricyclodecanedicarbaldehyde can be achieved by using the extraction solvent at a low volume ratio (extraction solvent/reaction product liquid).

When the concentration of the product is higher, a higher volume ratio (extraction solvent/reaction product liquid) is required to extract tricyclodecanedicarbaldehyde from the reaction product liquid.

When tricyclodecanedicarbaldehyde has relatively low solubility in the extraction solvent, the volume ratio (extraction solvent/reaction product liquid) may vary within the range of 10:1 to 1:10.

In order to extract a large amount of tricyclodecanedicarbaldehyde by using a small amount of the extraction solvent, it is effective to divide the extraction solvent to perform extraction operation several times. Further, in the final extraction operation, the hydroformylation reaction solvent such as methylcyclohexane may be added in an amount of about 5 to 20% by mass relative to the amount of the reaction product liquid. Addition of the hydroformylation reaction solvent makes it possible to increase a catalyst removal rate.

A temperature at which the extraction operation is performed is not limited, but from a practical viewpoint, the extraction operation is performed at a temperature equal to or less than the temperature of the hydroformylation reaction.

After the reaction, the extraction operation may be performed by adding the extraction solvent to the hydroformylation reactor or may be performed in an extraction tank after withdrawing the hydroformylation reaction product liquid from the hydroformylation reactor. When the extraction operation is performed by directly adding the extraction solvent to the hydroformylation reactor, the catalyst components may be retained in the hydroformylation reactor as it is to perform the next hydroformylation reaction. When the hydroformylation reaction product liquid is withdrawn to perform the operation in an extraction tank, the reaction solvent layer of the hydrocarbon compound, which contains the catalyst, is returned to the hydroformylation reactor to be used again for the reaction. This process can be performed either as a batch process or as a continuous process.

The above extraction operation makes it possible to obtain a tricyclodecanedicarbaldehyde-containing solution containing 10 to 90% by mass of tricyclodecanedicarbaldehyde and 10 to 90% by mass of the extraction solvent. When the reaction solvent is added, a tricyclodecanedicarbaldehyde-containing solution containing 5 to 90% by mass of tricyclodecanedicarbaldehyde, 5 to 90% by mass of the extraction solvent, and 5 to 90% by mass of the reaction solvent can be obtained.

The alcohol as an extraction solvent reacts with part of tricyclodecanedicarbaldehyde as a hydroformylation product to generate an acetal compound through acetalization of tricyclodecanedicarbaldehyde.

The content of the acetal compound in tricyclodecanedicarbaldehyde is usually about 0.1 to 50% by mass, particularly about 1 to 25% by mass.

<Hydrogenation Reduction Reaction>

The extraction liquid containing tricyclodecanedicarbaldehyde (tricyclodecanedicarbaldehyde-containing solution) obtained by the above extraction operation is then subjected to hydrogenation reduction in the presence of a publicly-known hydrogenation catalyst to produce TCDDM as shown by the following reaction formula (VI).

The hydrogenation reduction reaction is preferably performed in the presence of water and a hydrogenation catalyst because the acetal compound is rapidly converted to tricyclodecanedicarbaldehyde during the hydrogenation reaction of tricyclodecanedicarbaldehyde and the tricyclodecanedicarbaldehyde converted from the acetal compound is hydrogenated so that TCDDM can be produced at a high yield.

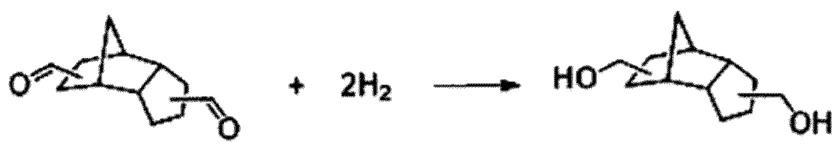
(VI)

The amount of water present in the hydrogenation reduction reaction is preferably an amount that is equal to or more than the amount of the acetal compound in a hydrogenation reduction reaction liquid and that does not cause phase separation of the reaction liquid. In the hydrogenation reduction reaction, the water content of the reaction liquid is 2% by mass or more, preferably 2 to 30% by mass, even more preferably 5 to 25% by mass, particularly preferably 10 to 20% by mass relative to the total mass of the reaction liquid. When the water content is within the above range, layer separation between water and the reaction solvent does not occur, and the above-described effect caused by the presence of water in the hydrogenation reaction system can effectively be obtained. Such water may be added in the extraction step in which the catalyst components and tricyclodecanedicarbaldehyde are separated from the hydroformylation reaction product liquid or may be added to the reaction system just before the hydrogenation reduction reaction.

The hydrogenation catalyst used in the hydrogenation reduction reaction is not limited and may be, for example, a ruthenium (Ru) catalyst or a nickel-supported diatomaceous earth catalyst.

The ruthenium (Ru) catalyst is not limited and may be, for example, commercially-available ruthenium (Ru)-supported carbon such as Ru/C catalyst (trade name, manufactured by N.E. CHEMCAT CORPORATION).

The nickel-supported diatomaceous earth catalyst is not limited and may be, for example, a nickel-supported diatomaceous earth catalyst disclosed in Example 1 in Japanese Patent Laid-Open No. 2005-279587 having a nickel content of 12% and a chromium content of 2%.

The reaction mode of the hydrogenation reduction reaction is appropriately selected, and a method in which a catalyst is charged as a slurry into a stirred reactor, a reaction is performed in a batchwise manner, and after the reaction, the catalyst is removed by sedimentation and filtration to be separated from a product liquid or a trickle-bed reaction in which a molded catalyst is charged into a tubular reactor and a product liquid and hydrogen gas are flowed on the catalyst may be adopted.

The amount of the catalyst to be used is not limited as long as TCDDM can be produced with industrially advantageous productivity.

The reaction temperature of the hydrogenation reduction reaction may be in the range of 125°C or more to 350°C or less from the viewpoint that the content of the chiral compound A (Xa/Xt) and the content of the chiral compound B (Xb/Xt) in a TCDDM composition to be obtained are controlled to fall within their respective desired ranges and the TCDDM composition of the present invention is efficiently produced.

Specifically, from the viewpoint of controlling the value of Xb/Xt of a TCDDM composition to be obtained to be 0.016 or more, the lower limit of reaction temperature of the hydrogenation reduction reaction is preferably 125°C or more, more preferably 130°C or more, even more preferably 140°C or more, particularly preferably 145°C or more, most preferably 150°C or more. On the other hand, from the viewpoint of preventing thermal decomposition and side reactions of TCDDM during the hydrogenation reduction reaction, the upper limit of the reaction temperature is preferably 350°C or less, more preferably 300°C or less, even more preferably 250°C or less, particularly preferably 200°C or less, most preferably 180°C or less.

The upper limit and the lower limit can freely be combined. For example, the reaction temperature of the hydrogenation reduction reaction is preferably 125°C or more and 350°C or less, more preferably 130°C or more and 300°C or less, even more preferably 140°C or more and 250°C or less, particularly preferably 145°C or more and 200°C or less, most preferably 150°C or more and 180°C or less.

The reaction pressure of the hydrogenation reduction reaction is not limited and may usually be 15 MPaG or less. The reaction pressure is preferably 10 MPaG or less, more preferably 7 MPaG or less because when the reaction pressure is increased, a pressure-tight reaction apparatus is required, which is economically inefficient. On the other hand, the lower limit of the reaction pressure is usually 1 MPaG.

<Removal of Residual Metal>

The crude reaction liquid obtained by the above hydrogenation reduction reaction operation contains a metal element derived from the hydrogenation catalyst as an eluted component. By removing the metal element from the crude reaction liquid prior to distillation purification, thermal decomposition of TCDDM caused by the metal element can be prevented in the distillation purification step.

A method for removing the metal element from the crude reaction liquid to reduce its content is not limited and may be activated carbon treatment, treatment with cation exchange resin, silica gel adsorption, or the like. From the viewpoint of removal efficiency and adsorbent reusability, activated carbon treatment is preferred.

The method of activated carbon treatment may be batch treatment in which activated carbon is added to the crude reaction liquid, the resultant is stirred, and the activated carbon is then removed by filtration or the like for solid-liquid separation or may be continuous treatment in which the crude reaction liquid is passed through an activated carbon-packed tower.

In the case of batch treatment, the amount of activated carbon added to the crude reaction liquid is appropriately determined by the ability of the activated carbon to adsorb metal elements, the metal element content of the crude reaction liquid, or the like. Generally, it is preferred that activated carbon is added at a concentration of about 0.01 to 10% by mass relative to the amount of the crude reaction liquid and the resultant is stirred.

In the case of continuous treatment, the flow rate of the crude reaction liquid to be treated is not limited, but the crude reaction liquid may be treated at a liquid hourly space velocity (LHSV) of 1 to 10.

Such activated carbon treatment may be performed twice or more. Specifically, an activated carbon-treated liquid obtained by treating the crude reaction liquid with activated carbon may again be treated with activated carbon. In this case, the type and amount of activated carbon used, treatment conditions, and the like may be changed between the first activated carbon treatment and the second activated carbon treatment.

The metal element content of the crude reaction liquid to be subjected to the subsequent distillation purification

step is preferably as low as possible from the viewpoint of preventing thermal decomposition of TCDDM. The metal element content of the crude reaction liquid to be subjected to distillation purification is preferably 10 ppm by mass or less, particularly preferably 5 ppm by mass or less, more particularly preferably 1 ppm by mass or less.

The pH of the crude reaction liquid to be subjected to distillation purification is preferably in the range of 6 to 8. The lower limit of the pH is preferably 6 or more because it is possible to prevent production of low-boiling compounds as by-products which is believed to be caused by dehydration of TCDDM or production of high-boiling compounds as by-products which is believed to be caused by dimerization such as etherification. The upper limit of the pH is preferably 8 or less because distillation purification equipment is less likely to be corroded by alkali.

The pH of the reaction product liquid obtained by the hydrogenation reduction reaction is usually 6 to 8 and is hardly changed even when the reaction product liquid is subjected to hydrogenation catalyst removal treatment or metal element removal treatment. However, the pH of the reaction product liquid may fall outside the range of 6 to 8 due to an acid or alkali component eluted from the hydrogenation catalyst. In this case, the pH is preferably adjusted to 6 to 8 by appropriately adding a pH adjusting agent such as an acid or an alkali.

<Distillation Purification>

The crude reaction liquid obtained by the above hydrogenation reduction reaction operation or the crude reaction liquid having a reduced metal element content obtained by the above residual metal removal operation is then subjected to distillation purification.

In the method for producing a tricyclodecanedimethanol composition of the present invention, the number and type of distillation towers used in the distillation purification step are not limited. For example, the distillation purification can be performed using a purification system equipped with one or two or more distillation towers. Specifically, the distillation purification can be performed using a combination of two or more appropriately selected from among a solvent separation distillation tower for removing a reaction solvent, a light-boiling separation distillation tower for removing by-products, impurities, and the like lower in boiling point than the TCDDM component, a high-boiling separation distillation tower for removing by-products, impurities, and the like higher in boiling point than the TCDDM component, and a thin-film distillation tower optionally provided for removing by-products, impurities, and the like higher in boiling point than the TCDDM component at a relatively low temperature in a short time.

From the viewpoint that the content of the chiral compound A (Xa/Xt) and the content of the chiral compound B (Xb/Xt) in a tricyclodecanedimethanol composition to be obtained is controlled to fall within their respective desired ranges and the tricyclodecanedimethanol composition of the present invention is efficiently produced, the distillation purification of the crude reaction liquid is preferably performed under conditions satisfying the following formula (1).

$$(Ya - Za) \times T/S \times 100 \leq 1.65 \quad (1)$$

(wherein

Ya: Mass ratio (unit: dimensionless number) of chiral compound A in crude reaction liquid supplied to distillation purification step
S: Total weight (unit: g) of chiral compound A, chiral compound B, chiral compound C, and chiral compound D in crude reaction liquid supplied to distillation purification step
Za: Mass ratio (unit: dimensionless number) of chiral compound A in distillate distilled off outside purification system from distillation tower in distillation purification step
T: Total weight (unit: g) of chiral compound A, chiral compound B, chiral compound C, and chiral compound D in distillate distilled off outside purification system from distillation tower in distillation purification step)

It should be noted that "distilled off outside purification system" herein means that a distillate at the bottom of the distillation tower and/or a distillate at the top of the distillation tower are discharged to the outside of the purification system.

When the distillation purification is performed using a purification system equipped with two or more distillation towers as described above, the values of Za and T may be values calculated based on the total amount of a distillate distilled off outside the purification system from these distillation towers.

In the above formula (1), (Ya - Za) is an index value indicating the ratio of the chiral compound A distilled off outside the purification system in the distillation purification step. When the value of (Ya - Za) is smaller, the value of Xa/Xt tends to be smaller due to a reduction in the amount of the chiral compound A contained in a TCDDM composition to be obtained.

In the above formula (1), T/S is an index value indicating the ratio of the low-boiling chiral compound B, chiral compound C, and chiral compound D distilled off outside the purification system as TCDDM in the distillation purification step. When the value of T/S is smaller, the value of Xa/Xt tends to be smaller due to a reduction in the amount of the chiral compound A contained in a TCDDM composition to be obtained.

Specifically, the value of (Ya - Za) × T/S × 100 represented in the above formula (1) is an index value indicating the discharge ratio of the chiral compound A distilled off (discharged) outside the purification system from the distillation tower in the distillation purification step. By using distillation conditions such that this value reduces, the content of the chiral compound A contained in a TCDDM composition to be obtained can be reduced to reduce the value of Xa/Xt.
From the viewpoint of controlling the value of Xa/Xt of a TCDDM composition to be obtained to be 0.430 or less, the upper limit of the value of (Ya - Za) × T/S × 100 is preferably 1.65 or less, more preferably 1.30 or less, even more preferably 1.00 or less, particularly preferably 0.65 or less, most preferably 0.3 or less. On the other hand, from the viewpoint of efficiently separating and removing by-products from the TCDDM composition, the lower limit of the value of (Ya - Za) × T/S × 100 is preferably 0.001 or more, more preferably 0.003 or more, even more preferably 0.010 or more, particularly preferably 0.015 or more, most preferably 0.030 or more. When the value of (Ya - Za) × T/S × 100 is smaller, it is more difficult to efficiently separate and remove by-products from the TCDDM composition so that the theoretical plate number and reflux ratio of the distillation tower need to be increased, which tends to be economically disadvantageous.
The upper limit and the lower limit can freely be combined. For example, the value of (Ya - Za) × T/S × 100 is preferably 0.001 or more and 1.65 or less, more preferably 0.003 or more and 1.30 or less, even more preferably 0.010 or more and 1.00 or less, particularly preferably 0.015 or more and 0.65 or less, most preferably 0.030 or more and 0.3 or less.
In the distillation purification, the bottom temperature of the distillation tower is not limited but may preferably be in the range of 150°C or more to 300°C or less.
When the lower limit of bottom temperature of the distillation tower is 150°C or more, TCDDM can efficiently be vaporized. The lower limit of bottom temperature of the distillation tower is preferably 160°C or more, more preferably 170°C or more, even more preferably 180°C or more. On the other hand, when the upper limit of bottom temperature of the distillation tower is 300°C or less, it is possible to prevent production of high-boiling impurities as by-products due to dimerization of TCDDM. The upper limit of bottom temperature of the distillation tower is preferably 280°C or less, more preferably 250°C or less, even more preferably 220°C or less.
The upper limit and lower limit of bottom temperature of the distillation tower can freely be combined.
The theoretical plate number of the distillation purification is not limited but may preferably be in the range of 5 or more to 45 or less.
When the lower limit of theoretical plate number of the distillation tower used for the distillation purification is 5 or more, impurities and a product can easily be separated. The lower limit of theoretical plate number of the distillation tower is more preferably 6 or more, even more preferably 7 or more, particularly preferably 8 or more, most preferably 10 or more. On the other hand, when the upper limit of theoretical plate number of the distillation tower is 45 or less, the difference in pressure between the top and bottom of the tower is small and the temperature at the bottom of the tower is low so that the heat load of the equipment is reduced. The upper limit of theoretical plate number of the distillation tower is more preferably 35 or less, even more preferably 25 or less, particularly preferably 15 or less, most preferably 13 or less.
The upper limit and lower limit of theoretical plate number of the distillation tower can freely be combined.
Other conditions of the distillation tower in the distillation purification are not limited, but the distillation purification is usually performed at a pressure of 0.1 kPaA or more and 100 kPaA or less and a reflux ratio of about 1 or more and 30 or less. Particularly, controlling the reflux ratio and the distillate amount makes it possible to adjust the abundance ratio among the chiral compound A, the chiral compound B, the chiral compound C, and the chiral compound D in a TCDDM composition to be obtained. These conditions may freely be changed depending on the equipment performance, pot efficiency, recovery amount, and the like of the distillation tower and are not limited as long as a TCDDM composition having a composition specified in the present invention can be obtained.
The bottom liquid of the distillation tower obtained by such distillation purification may further be subjected to simple distillation at 0.1 kPaA or more and 10 kPaA or less and 140°C or more and 250°C or less. Such distillation purification makes it possible to usually obtain a TCDDM composition having a TCDDM purity of 98% or more at a high yield.

<Ultraviolet-Curable Composition>

An ultraviolet-curable composition of the present invention is an ultraviolet-curable composition derived from the TCDDM composition of the present invention.
More specifically, the ultraviolet-curable composition of the present invention is an ultraviolet-curable composition synthesized using the TCDDM composition of the present invention as a raw material. Even more specifically, the ultraviolet-curable composition of the present invention is an ultraviolet-curable composition synthesized using tricyclodecanedimethanol contained in the TCDDM composition of the present invention as a raw material.
A method for producing the ultraviolet-curable composition is not limited and may be an ordinary method.
Generally, a derivative such as a di(meth)acrylic acid ester derivative or a urethane acrylate produced using tricyclodecanedimethanol in the TCDDM composition as a raw material is preferably used.
Both of the two hydroxyl groups of tricyclodecanedimethanol contained in the TCDDM composition may be used

for reaction or one of the hydroxyl groups may be reacted so that the other hydroxyl group remains. In this case, the remaining hydroxyl group may be converted to a functional group suitable for intended use by an organic synthesis technique, if necessary.

Specific examples of a method for producing a di(meth)acrylic acid ester derivative from TCDDM by using the TCDDM composition as a raw material include a method in which TCDDM is reacted with (meth)acrylic acid, a method in which TCDDM and a (meth)acrylic acid ester are subjected to a transesterification reaction, and a method in which TCDDM is reacted with a halide of (meth)acrylic acid such as (meth)acrylic acid chloride.

Reaction conditions and the like suitable for each of the above production methods are as follows.

When a di(meth)acrylic acid ester derivative is produced using (meth)acrylic acid or a (meth)acrylic acid ester, the reaction can be promoted by using a catalyst and continuously discharging water or a lower alcohol generated to the outside of the system.

Examples of the catalyst include publicly-known esterification catalysts such as sulfuric acid, para-toluenesulfonic acid, boron trifluoride, and an organotin compound, and any one selected from among these can be used.

The amount of the catalyst to be used is preferably 10 to 100000 ppm relative to the total mass of the reactive substrates from the viewpoint of, for example, reducing the load on production equipment and catalyst costs.

When a di(meth)acrylic acid ester derivative is produced using a halide of (meth)acrylic acid, the reaction is preferably performed in the presence of a basic compound and may also be promoted by using a catalyst.

Examples of the basic compound include publicly-known basic compounds such as tertiary amines such as triethylamine and N-ethyldiisopropylamine, phosphoric acid salts such as potassium phosphate and sodium phosphate, carbonates such as potassium carbonate and sodium carbonate, and hydroxides such as potassium hydroxide and sodium hydroxide, and any one selected from among these can be used.

The amount of the basic compound to be used is preferably 1.0 to 6.0 equivalents per equivalent of the halide of acrylic acid or methacrylic acid from the viewpoint of, for example, reducing the load on production equipment and raw material costs.

Examples of the catalyst include publicly-known catalysts for the esterification reaction of an acid chloride, such as pyridines such as N,N-dimethyl-4-aminopyridine, imidazoles such as N-methylimidazole, and tertiary amines such as triethylenediamine, and any one selected from among these can be used.

The amount of the catalyst to be used is preferably 10 to 100000 ppm relative to the total mass of the reactive substrates from the viewpoint of, for example, reducing the load on production equipment and catalyst costs.

In each of the above production methods, a polymerization inhibitor is preferably added to prevent thermal polymerization of the (meth)acrylic acid, the (meth)acrylic acid ester, or the halide of (meth)acrylic acid.

Examples of the polymerization inhibitor include hydroquinone, para-methoxyphenol, 2,4-dimethyl-6-t-butylphenol, 3-hydroxythiophenol, $\alpha$-nitroso-$\beta$-naphthol, para-benzoquinone, 2,5-dihydroxy-para-benzoquinone, copper salts, phenothiazine, paraphenylenediamine, and phenyl-$\beta$-naphthylamine.

The amount of the polymerization inhibitor to be used is preferably 10 to 100000 ppm relative to the total mass of the reactive substrates from the viewpoint of, for example, reducing influence on catalytic activity and side reactions.

The reaction temperature in each of the above production methods is preferably -20 to 120°C, more preferably 0 to 100°C from the viewpoint of reducing a reaction time and preventing polymerization. The reaction time is preferably 1 to 20 hours.

The reaction in each of the above production methods may be performed using a solvent.

The solvent is not limited as long as it does not adversely affect the reaction, and examples thereof include: aromatic hydrocarbons such as benzene, toluene, xylene, ethylbenzene, and mesitylene; aliphatic hydrocarbons such as pentane, hexane, heptane, octane, nonane, decane, cyclohexane, and cyclooctane; halogenated hydrocarbons such as dichloromethane, chloroform, 1,2-dichloroethane, carbon tetrachloride, chlorobenzene, and trifluoromethylbenzene; ethers such as diethyl ether, diisopropyl ether, dibutyl ether, anisole, tetrahydrofuran, and dioxane; ketones such as methyl ethyl ketone, diethyl ketone, and methyl isobutyl ketone; esters such as ethyl acetate and isopropyl acetate; nitriles such as acetonitrile; acyclic or cyclic amides such as dimethylformamide and N-methylpyrrolidinone; and acyclic or cyclic sulfoxides or sulfones such as dimethylsulfoxide. These solvents may be used singly or in combination of two or more of them.

The urethane acrylate produced using the TCDDM composition as a raw material is not limited by its structure as long as it is an acrylic acid or methacrylic acid ester derivative containing a urethane bond.

A specific method for producing a urethane acrylate from TCDDM by using the TCDDM composition as a raw material is preferably a method in which the TCDDM composition, a polyisocyanate compound, and a monohydroxyacrylate compound are reacted in the presence of a catalyst. For the purpose of adjusting the performance of a cured product, a polyol compound other than the TCDDM composition may further be added.

The mass ratio of the TCDDM composition to the total mass of the reactive substrates as urethane acrylate raw materials is preferably 10% by mass or more, more preferably 20% by mass or more, even more preferably 30% by mass or more from the viewpoint of hardness and heat resistance of a cured product. On the other hand, the upper limit of the mass

ratio is not limited and is preferably as high as possible.

Examples of the polyisocyanate compound include para-phenylene diisocyanate, 2,4-tolylenediisocyanate, 2,6-tolylenediisocyanate, 2,2'-dipehnylmethane diisocyanate, 2,4-diphenylmethane diisocyanate, 4,4'-diphenylmethane diisocyanate, diphenylether-4,4'-diisocyanate, o-xylylene diisocyanate, m-xylylene diisocyanate, p-xylylene diisocyanate, norbornane methane diisocyanate and their hydrides, pentamethylene diisocyanate, hexamethylene diisocyanate, dicyclohexylmethane diisocyanate, 1,4-cyclohexane diisocyanate, and isophorone diisocyanate. Nurates, adducts, and biurets of these compounds or dimers and trimers of these compounds may also be used. These may be used singly or in combination of two or more of them.

The monohydroxyacrylate compound is preferably one whose structural moiety that binds a hydroxy group and an acryloyloxy group is composed of three carbon atoms or more. Examples of such a compound include acrylates of aliphatic polyols having 3 or more carbon atoms, such as hydroxypropyl acrylate, trimethylolpropane diacrylate, pentaerythritol triacrylate, ditrimethylolpropane triacrylate, and dipentaerythritol pentaacrylate; (poly)oxyalkylene modified products obtained by introducing a (poly)oxyalkylene chain such as a (poly)oxyethylene chain, a (poly)oxypropylene chain, or a (poly)oxytetramethylene chain into the molecular structures of the above acrylate compounds; lactone modified products obtained by introducing a (poly)lactone structure into the molecular structures of the above acrylate compounds; isocyanuric acid diacrylate, (poly)oxyalkylene modified products obtained by introducing a (poly)oxyalkylene chain such as a (poly)oxyethylene chain, a (poly)oxypropylene chain, or a (poly)oxytetramethylene chain into the molecular structure of isocyanuric acid diacrylate; and lactone modified products obtained by introducing a (poly)lactone structure into the molecular structure of isocyanuric acid diacrylate. Alternatively, methacrylate compounds obtained by replacing acrylate in the above compounds with methacrylate may also be used. These may be used singly or in combination of two or more of them.

Examples of the polyol compound other than the TCDDM composition include: linear diols such as ethylene glycol, 1,3-propanediol, 1,4-butanediol, 1,5-pentanediol, and 1,6-hexanediol; branched diols such as 2-methyl-1,3-propanediol, 2,2-dimethyl-1,3-propanediol, 1,4-dimethylolhexane, 2-ethyl-1,3-hexanediol, 2,2,4-trimethyl-1,3-pentanediol, and dimer diols; diols having an ether group, such as diethylene glycol and propylene glycol; diols having an alicyclic structure, such as 1,4-cyclohexanediol, 1,4-cyclohexanedimethanol, and 1,4-dihydroxyethylcyclohexane; diols having an aromatic group, such as xylylene glycol, 1,4-dihydroxyethylbenzene, and 4,4'-methylenebis(hydroxyethylbenzene); polyols such as glycerin, trimethylolpropane, and pentaerythritol; polyether polyols, polyester polyols, and polycarbonate polyols. These polyol compounds may be used singly or in combination of two or more of them.

Examples of the catalyst include: organotin compounds such as dibutyltin dilaurate, trimethyltin hydroxide, and tetra-n-butyltin; organic bismuth compounds such as dibutyl bismuth dilaurate and dioctyl bismuth dilaurate; metallic salts such as zinc octylate, tin octylate, cobalt naphthenate, tin(II) chloride, and tin(IV) chloride; amine-based catalysts such as triethylamine, benzyldiethylamine, 1,4-diazabicyclo[2,2,2]octane, 1,8-diazabicyclo[5,4,0]undecene, N,N,N',N'-tetramethyl-1,3-butanediamine, and N-ethylmorpholine.

In order to prevent thermal polymerization of the monohydroxyacrylate compound and the like, a polymerization inhibitor is preferably added.

The polymerization inhibitor is not limited as long as it does not inhibit the reaction, and examples thereof include hydroquinone, para-methoxyphenol, 2,4-dimethyl-6-t-butylphenol, 3-hydroxythiophenol, $\alpha$-nitroso-$\beta$-naphthol, para-benzoquinone, 2,5-dihydroxy-para-benzoquinone, copper salts, phenothiazine, paraphenylenediamine, and phenyl-$\beta$-naphthylamine.

The amount of the polymerization inhibitor to be used is preferably 10 to 100000 ppm relative to the total mass of the reactive substrates from the viewpoint of, for example, reducing influence on catalytic activity and side reactions.

The reaction temperature in the above production method is preferably 30 to 120°C, more preferably 40 to 100°C from the viewpoint of reducing a reaction time and preventing polymerization. The reaction time is preferably 1 to 10 hours.

The reaction in each of the above production methods may be performed using a solvent.

The solvent is not limited as long as it does not adversely affect the reaction, and examples thereof include: aromatic hydrocarbons such as benzene, toluene, xylene, ethylbenzene, and mesitylene; aliphatic hydrocarbons such as heptane, octane, nonane, decane, cyclohexane, and cyclooctane; halogenated hydrocarbons such as dichloromethane, chloroform, 1,2-dichloroethane, carbon tetrachloride, chlorobenzene, and trifluoromethylbenzene; ethers such as diisopropyl ether, dibutyl ether, anisole, tetrahydrofuran, and dioxane; ketones such as methyl ethyl ketone, diethyl ketone, and methyl isobutyl ketone; esters such as ethyl acetate and isopropyl acetate; nitriles such as acetonitrile; acyclic or cyclic amides such as dimethylformamide and N-methylpyrrolidinone; and acyclic or cyclic sulfoxides or sulfones such as dimethylsulfoxide. These solvents may be used singly or in combination of two or more of them.

The ultraviolet-curable composition of the present invention is synthesized using the TCDDM composition of the present invention as a raw material and therefore has excellent coating stability without impairing performance originally required for the ultraviolet-curable composition. For this reason, the ultraviolet-curable composition of the present invention can suitably be used for hard coating materials, antifouling coating materials, resist materials, ink-jet inks, and materials for 3D printers.

When the ultraviolet-curable composition is used for resists, a method is used in which the ultraviolet-curable composition is applied onto a substrate by screen printing or flexographic printing and is then cured. In this case, the ultraviolet-curable composition is required to have excellent fluidity from the viewpoint of, for example, being able to apply it thinly and uniformly or thickly and being able to print a fine pattern on a substrate with high precision.
When the ultraviolet-curable composition is used for hard coats, antifouling coats, and ink-jet printing, a method is used in which the ultraviolet-curable composition is applied onto a substrate by a coater method, a spray method, or a dispenser method and is then cured. In this case, the ultraviolet-curable composition is required to have excellent fluidity from the viewpoint of accurately discharging a fixed amount of the ultraviolet-curable composition.
As described above, the ultraviolet-curable composition of the present invention is derived from the TCDDM composition of the present invention that has excellent storage stability and can maintain high fluidity even after long-term storage by appropriately controlling specific isomer ratios. This allows the ultraviolet-curable composition of the present invention to have excellent coating stability and application stability, which eliminates the need to add additives thereto. Therefore, the ultraviolet-curable composition of the present invention does not have such a problem that performance originally required for the ultraviolet-curable composition is impaired.
For these reasons, the ultraviolet-curable composition of the present invention can suitably be used for hard coating materials, antifouling coating materials, resist materials, ink-jet inks, and materials for 3D printers.

<Polymer Composition>

A polymer composition of the present invention is a polymer composition derived from the TCDDM composition of the present invention or a polymer composition derived from the ultraviolet-curable composition of the present invention.
More specifically, the polymer composition of the present invention is a polymer composition obtained by polymerizing the TCDDM composition of the present invention or a composition containing the TCDDM composition and is a composition containing a polymer having a structural unit derived from tricyclodecanedimethanol in the TCDDM composition. Alternatively, the polymer composition of the present invention is a composition containing a polymer obtained by polymerizing the ultraviolet-curable composition of the present invention.
An embodiment of the polymer composition of the present invention may be the polymer composition itself, a polymer composition obtained by further adding thereto or removing therefrom an appropriate amount of a solvent such as water or an organic solvent to adjust a solid content concentration, or a polymer composition obtained by removing therefrom the solvent and drying to form a solid. Alternatively, the embodiment may be a polymer composition obtained by purifying a composition containing a polymer obtained by polymerization through appropriate removal of impurities, and if necessary, appropriate additives such as a storage stabilizer (e.g., an ultraviolet absorber or an antioxidant), a colorant, an antistatic agent, a lubricant, a filler, a flame retarder, and a foaming agent may be added thereto without affecting the performance of the polymer obtained by polymerization.
That is, the polymer composition of the present invention is not limited by its form and component composition as long as it contains a polymer obtained by polymerizing the TCDDM composition of the present invention or the ultraviolet-curable composition of the present invention.
A specific example of the polymer constituting the polymer composition is at least one selected from the group consisting of a polyester-based resin, an epoxy-based resin, an acrylate-based resin, a polycarbonate-based resin, and a polyurethane-based resin.
In the present invention, the polyester-based resin is a resin mainly composed of a polyethylene-based polymer such as polyethylene terephthalate (PET). The polyethylene-based polymer is not limited and may be, for example, a polymer that contains a structural unit derived from a polyol mainly containing a glycol and a structural unit derived from a terephthalic acid-based compound and also contains a structural unit derived from tricyclodecanedimethanol in the TCDDM composition of the present invention.
In the present invention, the epoxy-based resin is a resin mainly composed of an epoxy-based polymer. The epoxy-based polymer is not limited and may be, for example, a polymer that contains a structural unit derived from a bisphenol-based compound and a structural unit derived from epichlorohydrin and also contains a structural unit derived from tricyclodecanedimethanol in the TCDDM composition of the present invention.
In the present invention, the acrylate-based resin is a resin mainly composed of an acrylate-based polymer. The acrylate-based polymer is not limited and may be, for example, a polymer that contains a structural unit derived from (meth) acrylic acid or a structural unit derived from a (meth)acrylic acid derivative and also contains a structural unit derived from tricyclodecanedimethanol in the TCDDM composition of the present invention.
In the present invention, the polycarbonate-based resin is a resin mainly composed of a polycarbonate-based polymer. The polycarbonate-based polymer is not limited and may be, for example, a polymer that contains a structural unit derived from a bisphenol compound, a structural unit derived from phosgene (carbonyl chloride), or a structural unit derived from diphenyl carbonate and also contains a structural unit derived from tricyclodecanedimethanol in the TCDDM composition of the present invention.

In the present invention, the polyurethane-based resin is a resin mainly composed of a polyurethane-based polymer. The polyurethane-based polymer is not limited and may be, for example, a polymer that contains a structural unit derived from a polyol mainly containing a glycol and a structural unit derived from a difunctional isocyanate and also contains a structural unit derived from tricyclodecanedimethanol in the TCDDM composition of the present invention.

## Examples

Hereinbelow, the present invention will more specifically be described with reference to examples. The present invention is not limited to these examples.
Compounds used in Examples and Comparative Examples are as follows.

- · Dicyclopentadiene (manufactured by FUJIFILM Wako Pure Chemical Corporation)
- · Acetylacetonato dicarbonyl rhodium (manufactured by N.E. CHEMCAT CORPORATION)
- · Tris(2,4-di-tert-butylphenyl)phosphite (manufactured by Tokyo Chemical Industry Co., Ltd.)
- · Methylcyclohexane (manufactured by FUJIFILM Wako Pure Chemical Corporation)
- · Ruthenium-supported carbon (Ru content 5% on dry basis, water content 56%) (trade name: Ru/C, manufactured by N.E. CHEMCAT CORPORATION)
- · Nickel-supported diatomaceous earth catalyst (nickel-supported diatomaceous earth having a nickel content of 12% and a chromium content of 2% self-produced according to Example 1 in Japanese Patent Laid-Open No. 2005-279587)
- · Copper oxide/aluminum oxide-containing catalyst (trade name: HySat, tablet size: 3 × 3 mm, manufactured by Clariant)

Evaluations in Examples and Comparative Examples were made by the following methods.

<Identification of Chiral Compounds A to D Contained in TCDDM Composition>

Chiral Compounds A to D contained in a TCDDM composition obtained in each of Examples and Comparative Examples were identified by nuclear magnetic resonance spectrometry according to the following procedure.
The TCDDM composition as a sample was dissolved in deuterochloroform ($CDCl_3$, containing 0.03 v/v% TMS), and the sample solution was transferred into an NMR sample tube having an outer diameter of 5 mm. Using a nuclear magnetic resonance spectrometer (trade name: AVANCE NEO 600-NMR, manufactured by Bruker), $^1$H-NMR, $^{13}$C-NMR, DEPT, COSY, TOCSY, NOESY, $^1$H-$^{13}$C HSQC, and $^1$H-$^{13}$C HMBC spectra of the sample solution were measured.
$^1$H-NMR was performed under measurement conditions of a resonant frequency of 600 MHz, a flip angle of 45°, a data acquisition time of 3 s, a pulse repetition time of 10 s, a number of scans of 16, and a measurement temperature of 25°C. Chemical shifts were referenced to the signal of TMS at 0.00 ppm.
$^{13}$C-NMR was performed under measurement conditions of a resonant frequency of 151 MHz, a flip angle of 45°, a data acquisition time of 2 s, a pulse repetition time of 5 s, a number of scans of 10000, and a temperature of 25°C. Chemical sifts were referenced to the signal of TMS at 0.00 ppm.
Structural identification was performed based on correlations between signals.

<Measurement of Xa, Xb, Xc, Xd, and Xt>

The number of moles of the chiral compound A, Xa, the number of moles of the chiral compound B, Xb, the number of moles of the chiral compound C, Xc, the number of moles of the chiral compound D, Xd, and the total number of moles of the chiral compound A, the chiral compound B, the chiral compound C, and the chiral compound D, Xt (= Xa + Xb + Xc + Xd) of each of the TCDDM compositions obtained in Examples and Comparative Examples were calculated by nuclear magnetic resonance spectrometry (NMR) according to the following procedure.
Sixty-five milligrams of the TCDDM composition as a sample was transferred into a sample tube, and 0.75 mL of deuterochloroform ($CDCl_3$, containing 0.03 v/v% TMS (tetramethylsilane)) was added thereto to obtain an NMR measurement sample.
It should be noted that addition of deuterochloroform to the sample of Comparative Example 1 yielded a white turbidity, and therefore 83 μL of deuteron-dimethylsulfoxide (DMSO-$d_6$, containing 0.05 v/v% TMS) was further added to the sample to obtain a transparent mixed solution of $CDCl_3$:DMSO-$d_6$ = 9:1, and such a mixed solution was used as an NMR measurement sample.
The NMR measurement sample was transferred into an NMR sample tube having an outer diameter of 5 mm, and a $^{13}$C-NMR spectrum was measured using a nuclear magnetic resonance spectrometer (trade name: AVANCE NEO 600-NMR, manufactured by Bruker).

$^{13}C$-NMR was performed under measurement conditions of a resonant frequency of 151 MHz, a flip angle of 45°, a data acquisition time of 2 s, a pulse repetition time of 5 s, a number of scans of 512, and a measurement temperature of 25°C. Chemical sifts were referenced to the signal of TMS at 0.00 ppm.

The $^{13}C$-NMR spectra of the TCDDM compositions obtained in Example 2 and Comparative Example 1 are respectively shown in Figure 2(a) and Figure 2(b).

The number of moles of each of the chiral compounds A, B, C, and D (Xa, Xb, Xc, Xd) and Xt (= Xa + Xb + Xc + Xd) were calculated using the total value of the signal intensity of carbon α on the norbornene ring in TCDDM and the signal intensity of carbon α on the norbornane ring in an enantiomer of the TCDDM as shown in the following structural formula (1).

It should be noted that only in the case of the chiral compound A of Comparative Example 1, the signal of carbon α on the norbornane ring overlapped with another signal, and therefore as shown in the following structural formula (2), Xa, Xb, Xc, Xd, and Xt were calculated using the total value of the signal intensity of carbon β on the norbornene ring in TCDDM and the signal intensity of carbon β on the norbornene ring in an enantiomer of the TCDDM.

In the $^{13}C$-NMR spectrum, the signals of carbon α on the norbornane ring of the chiral compounds A, B, C, and D were respectively observed in deuterochloroform at 27.9 ppm, 27.6 ppm, 28.4 ppm, and 27.2 ppm. Further, when deuteron-dimethylsulfoxide was added, the signals of carbon α on the norbornane ring of the chiral compounds A, B, C, and D were respectively observed at 27.8 ppm, 27.6 ppm, 28.3 ppm, and 27.4 ppm.

In the $^{13}C$-NMR spectrum, the signal of carbon β on the norbornane ring of the chiral compound A was observed at 30.7 ppm.

Using the obtained Xa, Xb, Xc, Xd, and Xt, Xa/Xt, Xb/Xt, Xc/Xt, Xd/Xt, and Xb/(Xa + Xc + Xd) were calculated.

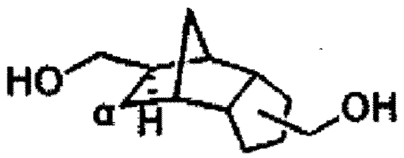

Structural Formula (1)

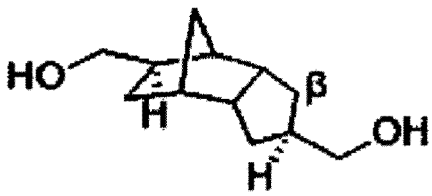

Structural Formula (2)

<Retention Times of Chiral Compound A and Chiral Compound B>

The retention times of the chiral compound A and the chiral compound B in each of the TCDDM compositions obtained in Examples and Comparative Examples were measured by gas chromatography (GC) under the following measurement conditions.

(Measurement Conditions)

Measurement device: Gas chromatograph (trade name: GC-2025, manufactured by Shimadzu Corporation)
Column: Capillary column (trade name: DB-1, manufactured by Agilent Technologies, length 30 m × inner diameter 0.25 mm × film thickness 1.00 μm)
Liquid phase: 100% Dimethylpolysiloxane
Carrier gas: Helium
Carrier gas column flow rate: 1 mL/min
Split ratio: 1/30
Sample volume: 0.3 μL
Oven temperature (heat-up conditions): 160°C (no holding time) → heating-up at 5°C/min → 300°C (2-min holding time)
Inlet temperature: 200°C
Ion source temperature: 300°C
Split ratio: 1/30

Detector: Flame ionization detector (temperature 300°C)

The gas chromatogram of the TCDDM composition obtained in Example 2 that will be described later is shown in Figure 1.

A peak marked with a label of (1) ("peak 1") between a retention time of 13.85 min and a retention time of 14.05 min is a peak of the chiral compound A in the TCDDM composition.

A peak marked with a label of (2) ("peak 2") between a retention time of 13.65 min and a retention time of 13.85 min is a peak of the chiral compound B in the TCDDM composition.

A peak marked with a label of (3) ("peak 3") between a retention time of 13.30 min and a retention time of 13.50 min is a peak of the chiral compound D in the TCDDM composition.

A peak marked with a label of (4) ("peak 4") between a retention time of 13.50 min and a retention time of 13.70 min is a peak of the chiral compound C in the TCDDM composition.

<Mass Analysis of TCDDM Composition>

In order to confirm generation of TCDDM, each of the TCDDM compositions obtained in Examples and Comparative Examples was subjected to gas chromatography-mass spectrometry according to the following procedure to measure m/z and a fragmentation pattern.

(Conditions of Gas Chromatography-Mass Spectrometry)

Measurement Device: Gas chromatograph (trade name: GCMS-QP2010 Ultra, manufactured by Shimadzu Corporation)
Carrier gas: Helium, linear velocity 40 cm/sec
Column: BPX-5 (manufactured by Trajan Scientific and Medical, length 60 m × inner diameter 0.32 mm × film thickness 0.25 μm)
Temperature (heat-up conditions): 160°C → heating-up at 5°C/min → 300°C (2-min holding time)
Vaporizing chamber temperature: 200°C
Ion source temperature: 250°C
MS interface temperature: 300°C
Injection volume: 0.5 μL
Split ratio: 1/30

<Evaluation of Storage Stability>

As an index of storage stability of each of the TCDDM compositions obtained in Examples and Comparative Examples, dynamic light scattering intensity of the TCDDM composition was measured by dynamic light scattering analysis according to the following procedure. Further, the TCDDM composition was visually observed during the measurement to evaluate fluidity.

A glass sample bottle containing the TCDDM composition as a sample was immersed in an oil bath set to a temperature of 60°C, and the sample was heated with stirring until the measured temperature of the TCDDM composition was increased to 60°C. Then, the stirring of the sample was stopped, 1 mL of the TCDDM composition was sampled and placed in a measurement cell, the measurement cell was placed in a thermostatic bath included in a dynamic light scattering analyzer, and measurement of dynamic light scattering intensity and visual observation for evaluation of fluidity were performed under the following measurement conditions while the measured temperature of the TCDDM composition was maintained at 60°C.

It should be noted that the measurement of dynamic light scattering intensity and the visual observation for evaluation of fluidity were performed just before the TCDDM composition sample was immersed in the oil bath (0 hours) and after 96 hours, 175 hours, and 196 hours from the start of the immersion in the oil bath.

(Measurement Conditions)

Measurement device: Dynamic light scattering analyzer (trade name: Zetasizer Nano ZS, manufactured by Malvern Panalytical)

Detection method: 173° backscatter detection (A scattered light detector was placed at the back of the sample at an angle of 173°.)
Sample temperature: 60°C
Cell: Glass

Further, storage stability of the TCDDM composition was evaluated according the following criteria.

It should be noted that fluidity was evaluated in the following manner. In a state where the glass sample bottle was immersed in the thermostatic bath of the dynamic light scattering analyzer, the tip of a glass rod was dipped 10 mm below the surface of the TCDDM composition sample in the sample bottle, and then the glass rod was raised by 10 cm. At this time, when exhibiting stringiness, the TCDDM composition was evaluated as "with fluidity" and when not exhibiting stringiness, the TCDDM composition was evaluated as "without fluidity".

(Evaluation Criteria)

A: The TCDDM composition exhibited fluidity after 196 hours, and the dynamic light scattering intensity was 3500 or less.
B: The TCDDM composition exhibited fluidity after 196 hours, and the dynamic light scattering intensity exceeded 3500.
C: The TCDDM composition exhibited fluidity after 96 hours but did not exhibit fluidity after 175 hours, and the dynamic light scattering intensity exceeded 100000.
D: The TCDDM composition did not exhibit fluidity after 96 hours, and the dynamic light scattering intensity exceeded 100000.

[Example 1]

<Hydroformylation Reaction>

In an atmosphere of nitrogen, 11 mg of acetylacetonato dicarbonyl rhodium and 870 mg of tris(2,4-di-tert-butylphenyl)phosphite were weighed as raw material compounds of a hydroformylation reaction catalyst, and these raw material compounds and 69 g of methylcyclohexane and 89 g of dicyclopentadiene as organic solvents were charged one after another into an autoclave reactor (up-and-down stirring type) having an internal capacity of 500 mL. Then, a reaction liquid in the reactor was heated to 70°C while being stirred. Then, a mixed gas of hydrogen and carbon monoxide (hydrogen:carbon monoxide = 1:1 (mole ratio)) was quickly introduced under pressure through a gas inlet valve so that the pressure in the reactor was 3 MPaG to perform a reaction for 1 hour. Then, the temperature of the reaction liquid was increased to 100°C and then a reaction was further performed for 5 hours. During the reaction, the mixed gas was continuously introduced into the reactor in an amount consumed by the reaction while the pressure in the reactor was maintained at 3.0 MPaG.

After the completion of the reaction, the reaction liquid in the reactor was cooled to room temperature and the gas remaining in the reactor was released to obtain 192 g of a hydroformylation reaction product liquid. The amount of dicyclpentadiene contained as a raw material compound in the reaction liquid before the reaction and the amount of tricyclodecanedicarbaldehyde contained as a product in the hydroformylation reaction product liquid after the reaction were analyzed by gas chromatography to determine the yield of tricyclodecanedicarbaldehyde. The yield was 99%.

<Extraction Operation>

Fifty-one grams of methanol and 35 g of water were added to 172 g of the obtained hydroformylation reaction product liquid, and the resultant was stirred for 30 minutes in an atmosphere of nitrogen. Then, the resultant was left to stand for 30 minutes to be separated into two phases, and extraction operation was performed to obtain a lower phase (a1). Then, 6.6 g of methylcyclohexane was added to the lower phase (a1), and the resultant was stirred for 30 minutes. Then, the resultant was left to stand for 30 minutes to be separated into two phases, and extraction operation was performed to obtain 202.3 g of a lower phase (a2).

The obtained lower phase (a2) was analyzed by gas chromatography and was found to have a composition of 52% tricyclodecanedicarbaldehyde, 27% methanol, 14% water, 2% methylcyclohexane, and 5% other components by mass.

<Hydrogenation Reduction Reaction>

In an autoclave reactor having an internal capacity of 500 mL, 200 g of the lower phase (a2) obtained by the above-described extraction operation and 0.6 g of ruthenium-supported carbon as a hydrogenation reduction reaction catalyst were charged. Then, a reaction liquid in the reactor was heated to 160°C while being stirred at 1200 rpm. Then, hydrogen gas was introduced under pressure through a gas inlet valve so that the pressure in the reactor was 3 MPaG, and a reaction was performed for 3 hours while the pressure in the reactor and the temperature of the reaction liquid were maintained. During the reaction, the mixed gas was continuously introduced into the reactor in an amount consumed by the reaction so that the pressure in the reactor was maintained at 3 MPaG.

After the completion of the reaction, the reaction liquid in the reactor was cooled to room temperature, the gas remaining in the reactor was released, and the ruthenium-supported carbon was removed by filtration using a filter having a pore diameter of 5 μm to obtain 176 g of a reaction product liquid. The amount of tricyclodecanedicarbaldehyde contained as a raw material compound in the reaction liquid before the reaction and the amount of TCDDM contained as a product in the reaction product liquid after the reaction were analyzed by gas chromatography. The yield of TCDDM was 98%.

<Distillation Purification>

First, 170 g of the reaction product liquid after the hydrogenation reduction reaction was charged into a four-necked flask with an internal capacity of L equipped with a batch-type distillation tower packed with structured packings equivalent to five theoretical plates, and 80.2 g of a light-boiling component mainly containing a solvent was distilled off at a minimum pressure in the tower of 10 kPaA and a maximum bottom temperature of the tower of 100°C. Then, the pressure in the tower was set to 0.3 kPaA and the bottom temperature of the tower was set to 120°C, and distillation was performed until 1.5 g of a distillate was obtained from the top of the distillation tower.

Then, the distillation tower packed with structured packings equivalent to five theoretical plates was replaced with a glass single distillation tower, and simple distillation was performed at a pressure of 0.3 kPaA and a temperature of 165°C to distill off a TCDDM composition from the top of the tower to collect an initial distillate and a final distillate in this order. The amount of the TCDDM composition collected as a final distillate was 64.6 g.

The values of S, T, Ya, Za, and (Ya - Za) × (T/S) × 100 in this distillation purification are shown in Table 1.

The values of Xa/Xt, Xb/Xt, Xc/Xt, Xd/Xt, and Xb/(Xa + Xc + Xd) and storage stability of the obtained TCDDM composition were evaluated. The evaluation results are shown in Table 2.

[Example 2]

A hydrogenation reduction reaction was performed under the same conditions as in Example 1 except that a nickel-supported diatomaceous earth catalyst was used instead of the ruthenium-supported carbon, and as a result, 175 g of a reaction product liquid was obtained. The yield of TCDDM obtained by the hydrogenation reduction reaction was 99%.

Then, 170 g of the reaction product liquid after the hydrogenation reduction reaction was subjected to distillation purification under the same conditions as in Example 1 except that the values of S, T, Ya, Za, and (Ya - Za) × (T/S) × 100 were changed as shown in Table 1. The amount of a TCDDM composition collected as a final distillate after the distillation purification was 754 g.

The evaluation results of the obtained TCDDM composition are shown in Table 2.

[Example 3]

A hydrogenation reduction reaction was performed under the same conditions as in Example 1 except that the temperature of the reaction liquid was changed from 160°C to 180°C and that a nickel-supported diatomaceous earth catalyst was used instead of the ruthenium-supported carbon, and as a result, 176 g of a reaction product liquid was obtained. The yield of TCDDM obtained by the hydrogenation reduction reaction was 99%.

Then, 170 g of the reaction product liquid after the hydrogenation reduction reaction was subjected to distillation purification under the same conditions as in Example 1 except that the values of S, T, Ya, Za, and (Ya - Za) × (T/S) × 100 were changed as shown in Table 1. The amount of a TCDDM composition collected as a final distillate after the distillation purification was 65.3 g.

The evaluation results of the obtained TCDDM composition are shown in Table 2.

[Example 4]

A hydrogenation reduction reaction was performed under the same conditions as in Example 1 except that a

copper oxide/aluminum oxide-containing catalyst was used instead of the ruthenium-supported carbon, and as a result, 174 g of a reaction product liquid was obtained. The yield of TCDDM obtained by the hydrogenation reduction reaction was 99%.

Then, 170 g of the reaction product liquid after the hydrogenation reduction reaction was distilled until 11.6 g of a distillate was obtained from the top of a distillation tower in the same manner as the distillation purification in Example 1 except that a distillation tower packed with structured packings equivalent to 15 theoretical plates was attached to the four-necked flask with an internal capacity of 3 L instead of the batch-type distillation tower packed with structured packings equivalent to 5 theoretical plates and that the pressure in the tower was set to 0.6 kPaA and the bottom temperature of the tower was set to 185°C after a light-boiling component mainly containing a solvent was distilled off.

Then, the distillation tower packed with structured packings equivalent to 15 theoretical plates was replaced with a glass single distillation tower to perform simple distillation under the same conditions as in Example 1. The amount of a TCDDM composition collected as a final distillate after the simple distillation was 53.0 g.

The values of S, T, Ya, Za, and (Ya - Za) × (T/S) × 100 in this distillation purification are shown in Table 1.

The evaluation results of the obtained TCDDM composition are shown in Table 2.

[Comparative Example 1]

One hundred and seventy grams of the reaction product liquid after the hydrogenation reduction reaction was distilled until 11.7 g of a distillate was obtained from the top of a distillation tower in the same manner as the distillation purification in Example 1 except that a distillation tower packed with structured packings equivalent to 20 theoretical plates was attached to the four-necked flask with an internal capacity of 3 L instead of the batch-type distillation tower packed with structured packings equivalent to 5 theoretical plates and that the pressure in the tower was set to 0.6 kPaA and the bottom temperature of the tower was set to 185°C after a light-boiling component mainly containing a solvent was distilled off. Then, the distillation tower packed with structured packings equivalent to 20 theoretical plates was replaced with a glass single distillation tower to perform simple distillation under the same conditions as in Example 1. The amount of a TCDDM composition collected as a final distillate after the simple distillation was 54.8 g.

The values of S, T, Ya, Za, and (Ya - Za) × (T/S) × 100 in this distillation purification are shown in Table 1.

The evaluation results of the obtained TCDDM composition are shown in Table 2.

[Comparative Example 2]

A hydrogenation reduction reaction was performed under the same conditions as in Example 1 except that the temperature of the reaction liquid was changed from 160°C to 120°C, and as a result, 177 g of a reaction product liquid was obtained. The yield of TCDDM obtained by the hydrogenation reduction reaction was 98%.

Then, 170 g of the reaction product liquid after the hydrogenation reduction reaction was subjected to distillation purification under the same conditions as in Example 1 except that the values of S, T, Ya, Za, and (Ya - Za) × (T/S) × 100 were changed as shown in Table 1. The amount of a TCDDM composition collected as a final distillate after the simple distillation was 65.1 g.

The evaluation results of the obtained TCDDM composition are shown in Table 2.

[Comparative Example 3]

A hydroformylation reaction was performed to obtain a hydroformylation reaction product liquid under the same conditions as in Example 1 except that the amount of tris(2,4-di-tert-butylphenyl)phosphite was changed from 870 mg to 1450 mg and that the pressure in the reactor was changed from 3.0 MPaG to 5.0 MPaG. The yield of obtained tricyclodecanedicarbaldehyde was 98%.

Then, 193 g of the obtained hydroformylation reaction product liquid was subjected to extraction operation, hydrogenation reduction reaction, and distillation purification under the same conditions as in Example 1 except that the values of S, T, Ya, Za, and (Ya - Za) × (T/S) × 100 in the distillation purification were changed as shown in Table 1. The amount of a TCDDM composition collected as a final distillate after the simple distillation was 63.6 g.

The evaluation results of the obtained TCDDM composition are shown in Table 2.

Each of the TCDDM compositions obtained in Examples 1 to 4 and Comparative Examples 1 to 3 was analyzed by gas chromatography-mass spectrometry in such a manner as described above to measure m/z and a fragmentation pattern. As a result, all of the TCDDM compositions were confirmed to have peaks 1 to 3 showing m/z values and a fragmentation pattern corresponding to those of TCDDM.

Typical fragments observed are shown below.

MS(EI): 178([M-18]+), 165, 147, 119, 105, 91, 81, 67

[Table 1]

| | | Unit | Example 1 | Example 2 | Example 3 | Example 4 |
|---|---|---|---|---|---|---|
| Reaction pressure of hydroformylation | | MPaG | 3.0 | 3.0 | 3.0 | 3.0 |
| Temperature of hydrogen reduction reaction | | °C | 160 | 160 | 180 | 160 |
| (Ya - Za) × (T/S) × 100 of distillation tower | | - | 0.025 | 0.021 | 0.021 | 1.20 |
| | S | g | 88.4 | 88.4 | 88.2 | 88.4 |
| | T | g | 0.18 | 0.18 | 0.18 | 10.3 |
| | Ya | - | 0.386 | 0.350 | 0.345 | 0.279 |
| | Za | - | 0.262 | 0.246 | 0.240 | 0.176 |

| | | Unit | Comparative Example 1 | Comparative Example 2 | Comparative Example 3 |
|---|---|---|---|---|---|
| Reaction pressure of hydroformylation | | MPaG | 3.0 | 3.0 | 5.0 |
| Temperature of hydrogen reduction reaction | | °C | 160 | 120 | 160 |
| (Ya - Za) × (T/S) × 100 of distillation tower | | - | 1.73 | 0.025 | 0.026 |
| | S | g | 88.4 | 88.4 | 88.4 |
| | T | g | 10.2 | 0.18 | 0.18 |
| | Ya | - | 0.386 | 0.413 | 0.444 |
| | Za | - | 0.236 | 0.290 | 0.315 |

[Table 2]

| | | Unit | Example 1 | Example 2 | Example 3 | Example 4 |
|---|---|---|---|---|---|---|
| Component ratio of TCDDM composition | Xa/Xt | - | 0.367 | 0.344 | 0.338 | 0.411 |
| | Xb/Xt | - | 0.018 | 0.028 | 0.035 | 0.060 |
| | Xc/Xt | - | 0.334 | 0.336 | 0.336 | 0.306 |
| | Xd/Xt | - | 0.281 | 0.292 | 0.291 | 0.218 |
| | Xb/(Xa+Xc+Xd) | - | 0.019 | 0.029 | 0.036 | 0.064 |
| Retention time in GC measurement | Compound A | min | 13.90 | 13.90 | 13.90 | 13.90 |
| | Compound B | min | 13.75 | 13.75 | 13.75 | 13.75 |
| Storage stability (upper: dynamic light scattering intensity, lower: fluidity) | 0 hours | - | 749 with fluidity | 587 with fluidity | 841 with fluidity | 696 with fluidity |
| | 96 hours | - | 3868 with fluidity | 3135 with fluidity | 2360 with fluidity | 3182 with fluidity |
| | 175 hours | - | 528304 without fluidity | - with fluidity | - with fluidity | 837007 without fluidity |
| | 196 hours | - | without fluidity | 4220 with fluidity | 3006 with fluidity | without fluidity |
| | Evaluation | - | C | B | A | C |

| | | Unit | Comparative Example 1 | Comparative Example 2 | Comparative Example 3 |
|---|---|---|---|---|---|
| Component ratio of TCDDM composition | Xa/Xt | - | 0.445 | 0.409 | 0.436 |
| | Xb/Xt | - | 0.050 | 0.009 | 0.019 |
| | Xc/Xt | - | 0.284 | 0.310 | 0.292 |
| | Xd/Xt | - | 0.220 | 0.272 | 0.252 |
| | Xb/(Xa+Xc+Xd) | - | 0.053 | 0.005 | 0.010 |
| Retention time in GC measurement | Compound A | min | 13.90 | 13.90 | 13.90 |
| | Compound B | min | 13.75 | 13.75 | 13.75 |
| Storage stability (upper: dynamic light scattering intensity, lower: fluidity) | 0 hours | - | 64 with fluidity | 192 with fluidity | 1779 with fluidity |
| | 96 hours | - | 208618 without fluidity | 489540 without fluidity | - without fluidity |
| | 175 hours | - | without fluidity | without fluidity | without fluidity |
| | 196 hours | - | - without fluidity | - without fluidity | - without fluidity |
| | Evaluation | - | D | D | D |

In the evaluation of storage stability, a lower analytical value of dynamic light scattering intensity indicates that the TCDDM composition has lower crystallinity and higher fluidity, that is, the TCDDM composition has excellent storage stability.

As can be seen from Table 1, the TCDDM compositions obtained in Examples 1 to 4 were superior in storage stability to the TCDDM compositions of Comparative Examples 1 to 3. Particularly, the TCDDM compositions of Example 2 and Example 3 were much superior in storage stability.

The TCDDM compositions obtained in Comparative Examples 1 and 3 were poor in storage stability due to their large Xa/Xt values.

The TCDDM composition obtained in Comparative Example 2 was poor in storage stability due to its small Xb/Xt value.

The present invention has been described above in detail with reference to specific aspects, but it is apparent to those skilled in the art that various modifications can be made without departing from the intent and scope of the present invention.

The present application is based on Japanese Patent Application No. 2023-148245 filed on September 13, 2023 and Japanese Patent Application No. 2024-017280 filed on February 7, 2024, the disclosures of which are incorporated herein by reference in their entirety.

## Claims

**1.** A tricyclodecanedimethanol composition comprising a chiral compound A, one of enantiomers of which is represented by the following formula (I), a chiral compound B, one of enantiomers of which is represented by the following formula (II), a chiral compound C, one of enantiomers of which is represented by the following formula (III), and a chiral compound D, one of enantiomers of which is represented by the following formula (IV), wherein
a number of moles of the chiral compound A, Xa and a number of moles of the chiral compound B, Xb as measured by nuclear magnetic resonance spectrometry and a total number of moles of the chiral compound A, the chiral compound B, the chiral compound C, and the chiral compound D, Xt satisfy Xa/Xt ≤ 0.430 and Xb/Xt ≥ 0.016:

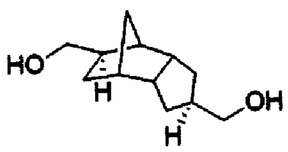
(I)

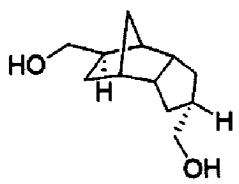
(II)

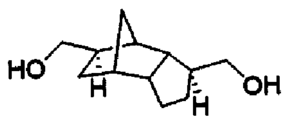 (III) 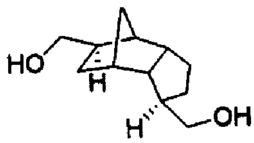 (IV)

**2.** The tricyclodecanedimethanol composition according to claim 1, wherein the Xb and the Xt satisfy $Xb/Xt \geq 0.020$.

**3.** The tricyclodecanedimethanol composition according to claim 1, wherein the Xa and the Xt satisfy $Xa/Xt \leq 0.400$.

**4.** The tricyclodecanedimethanol composition according to claim 1, wherein the Xb and the Xt satisfy $Xb/Xt \geq 0.027$.

**5.** The tricyclodecanedimethanol composition according to claim 1, wherein the Xa and the Xt satisfy $Xa/Xt < 0.350$.

**6.** The tricyclodecanedimethanol composition according to claim 1, wherein the number of moles of the chiral compound C, Xc as measured by nuclear magnetic resonance spectrometry and the Xt satisfy $Xc/Xt \geq 0.300$.

**7.** The tricyclodecanedimethanol composition according to claim 1, wherein the number of moles of the chiral compound D, Xd as measured by nuclear magnetic resonance spectrometry and the Xt satisfy $Xd/Xt \geq 0.240$.

**8.** The tricyclodecanedimethanol composition according to claim 1, wherein the Xa, the Xb, and the number of moles of the chiral compound C, Xc and the number of moles of the chiral compound D, Xd as measured by nuclear magnetic resonance spectrometry satisfy $Xb/(Xa + Xc + Xd) \geq 0.010$.

**9.** The tricyclodecanedimethanol composition according to claim 1, wherein the chiral compound B is detected at a retention time of 13.65 to 13.85 minutes when the composition is measured by gas chromatography (GC) under the following measurement conditions:

(Measurement Conditions)
Column: Capillary column (length 30 m × inner diameter 0.25 mm × film thickness 1 μm)
Liquid phase: 100% Dimethylpolysiloxane
Carrier gas: Helium
Carrier gas column flow rate: 1 mL/min
Split ratio: 1/30
Injection volume: 0.3 μL
Oven temperature: 160°C (no holding time) → heating-up at 5°C/min → 300°C (2-min holding time)
Inlet temperature: 200°C
Detector: Flame ionization detector (temperature 300°C)

**10.** The tricyclodecanedimethanol composition according to claim 9, wherein the chiral compound A is detected at a retention time of 13.85 to 14.05 minutes when the composition is measured by gas chromatography (GC) under the measurement conditions.

**11.** An ultraviolet-curable composition derived from the tricyclodecanedimethanol composition according to any one of claims 1 to 10.

**12.** The ultraviolet-curable composition according to claim 11, which is used for any one of a hard coating material, an antifouling coating material, a resist material, an ink-jet ink, and a material for 3D printers.

**13.** A polymer composition derived from the tricyclodecanedimethanol composition according to any one of claims 1 to 10.

**14.** The polymer composition according to claim 13, which comprises, as a polymer, at least one selected from the group consisting of a polyester-based resin, an epoxy-based resin, an acrylate-based resin, a polycarbonate-based resin, and a polyurethane-based resin.

**15.** A polymer composition derived from the ultraviolet-curable composition according to claim 11.

**16.** The polymer composition according to claim 15, which comprises, as a polymer, at least one selected from the group

consisting of a polyester-based resin, an epoxy-based resin, an acrylate-based resin, a polycarbonate-based resin, and a polyurethane-based resin.

**17.** A method for producing a tricyclodecanedimethanol composition, the method comprising the steps of:

subjecting dicyclopentadiene to hydroformylation to obtain tricyclodecanedicarbaldehyde;
subjecting the tricyclodecanedicarbaldehyde to a reduction reaction to obtain a crude reaction liquid containing tricyclodecanedimethanol; and
subjecting the crude reaction liquid to distillation purification to obtain the tricyclodecanedimethanol composition according to any one of claims 1 to 10.

**18.** The method for producing a tricyclodecanedimethanol composition according to claim 17, wherein

a reaction pressure of the hydroformylation is 0.5 MPaG or more and 4.5 MPaG or less,
a temperature of the reduction reaction is 125°C or more and 350°C or less, and
distillation conditions of the distillation purification satisfy the following formula (1):

$$(Ya - Za) \times T/S \times 100 \leq 1.65 \quad (1)$$

(wherein
Ya: Mass ratio (unit: dimensionless number) of chiral compound A in crude reaction liquid supplied to distillation purification step
S: Total weight (unit: g) of chiral compound A, chiral compound B, chiral compound C, and chiral compound D in crude reaction liquid supplied to distillation purification step
Za: Mass ratio (unit: dimensionless number) of chiral compound A in distillate distilled off outside purification system from distillation tower in distillation purification step
T: Total weight (unit: g) of chiral compound A, chiral compound B, chiral compound C, and chiral compound D in distillate distilled off outside purification system from distillation tower in distillation purification step).

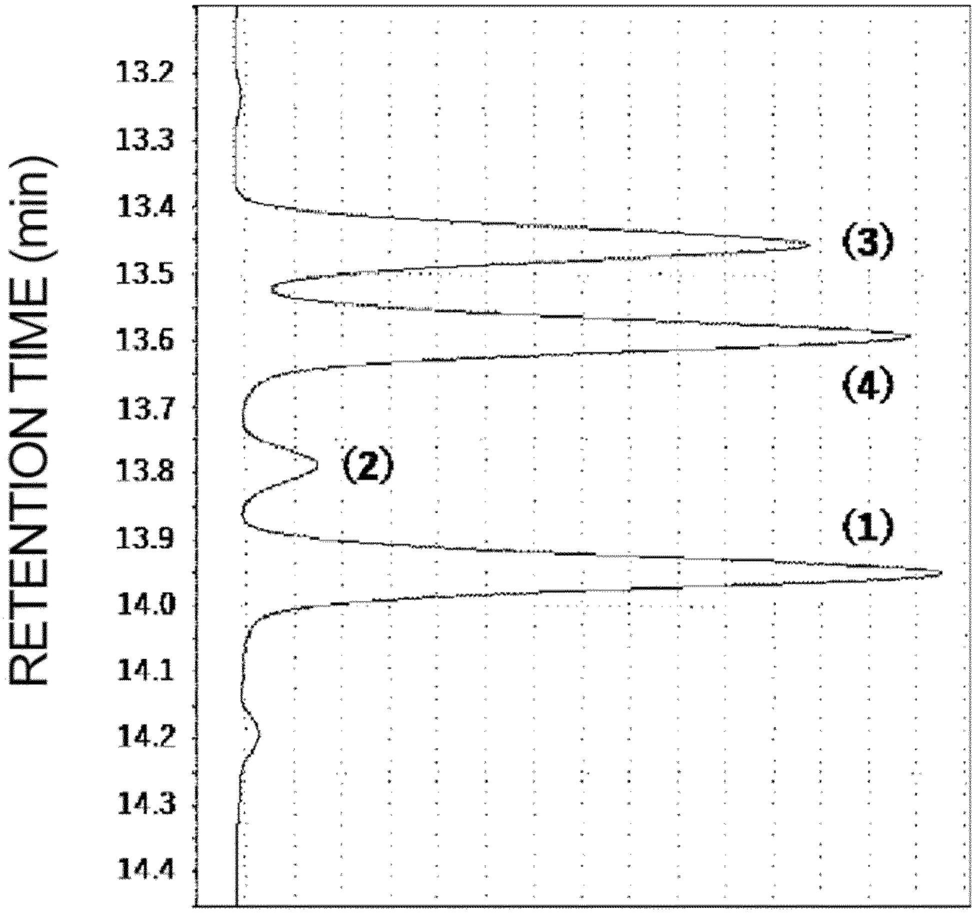
RETENTION TIME (min)
13.2
13.3
13.4
13.5
13.6
13.7
13.8
13.9
14.0
14.1
14.2
14.3
14.4
(3)
(4)
(2)
(1)

Fig. 1

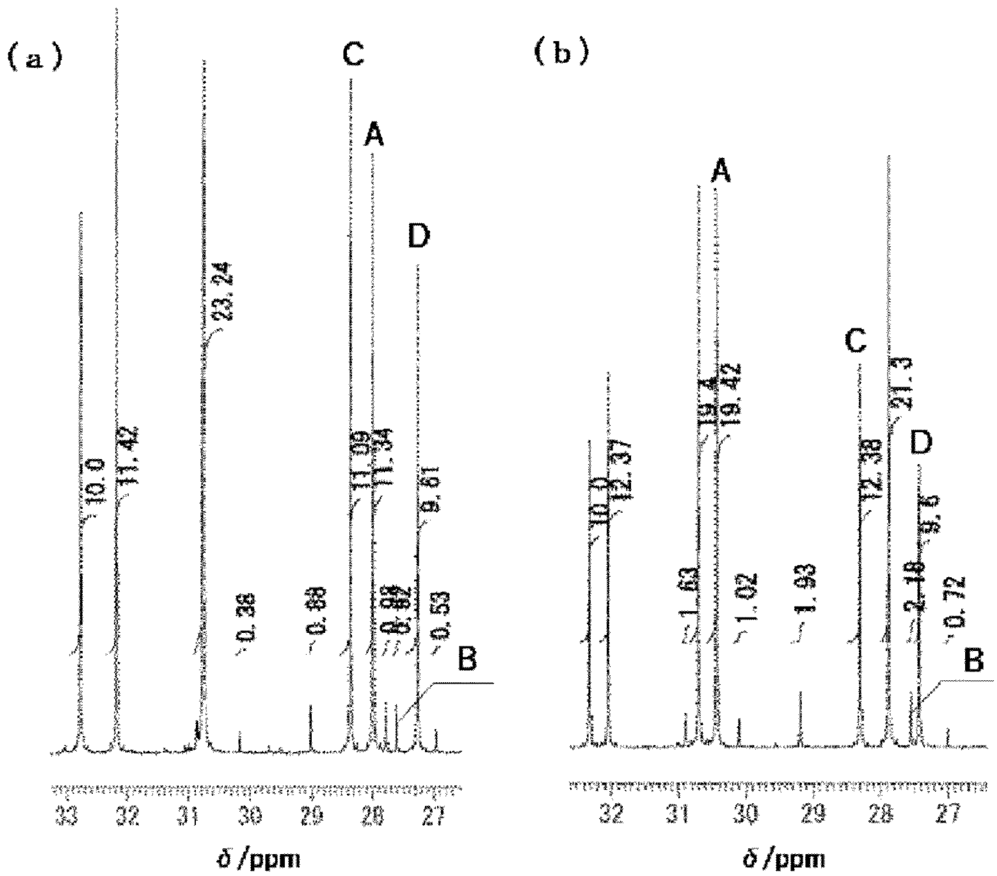
(a)
C
A
D
B
10.0
11.42
23.24
0.38
0.88
11.09
11.34
9.61
0.53
33
32
31
30
29
28
27
δ /ppm
(b)
A
C
D
B
10.0
12.37
19.4
19.42
1.63
1.02
1.93
12.38
21.3
9.6
2.18
0.72
32
31
30
29
28
27
δ /ppm

Fig. 2

**INTERNATIONAL SEARCH REPORT**

International application No. **PCT/JP2024/032495**

**A. CLASSIFICATION OF SUBJECT MATTER**

***C07C 31/27***(2006.01)i; ***C07B 61/00***(2006.01)i; ***C07C 29/141***(2006.01)i; ***C07C 45/50***(2006.01)i; ***C07C 47/347***(2006.01)i; ***C08F 20/10***(2006.01)i; ***C08G 18/32***(2006.01)i; ***C08G 59/24***(2006.01)i; ***C08G 63/123***(2006.01)i; ***C08G 64/02***(2006.01)i; ***C09D 11/30***(2014.01)i; ***C09D 201/02***(2006.01)i

FI: C07C31/27; C07C29/141; C09D201/02; C09D11/30; C08G63/123; C08G64/02; C08G59/24; C08F20/10; C08G18/32 012; C07C45/50; C07C47/347; C07B61/00 300

According to International Patent Classification (IPC) or to both national classification and IPC

**B. FIELDS SEARCHED**

Minimum documentation searched (classification system followed by classification symbols)

C07C31/27; C07B61/00; C07C29/141; C07C45/50; C07C47/347

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2023/277347 A1 (SK CHEMICALS CO., LTD.) 05 January 2023 (2023-01-05) claims, paragraphs [0032]-[0065], examples | 1-18 |
| A | WO 2022/265240 A1 (SK CHEMICALS CO., LTD.) 22 December 2022 (2022-12-22) claims, paragraphs [0097]-[0140], examples | 1-18 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 October 2024** | **29 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**
**Information on patent family members**

International application No.
**PCT/JP2024/032495**

| Patent document cited in search report | Publication date (day/month/year) | Patent family member(s) | Publication date (day/month/year) |
|---|---|---|---|
| WO 2023/277347 A1 | 05 January 2023 | US 2023/0279243 A1<br>claims, paragraphs [0019]-[0033], examples<br>TW 202317507 A<br>KR 10-2435355 B1 | |
| WO 2022/265240 A1 | 22 December 2022 | US 2023/0192579 A1<br>claims, paragraphs [0048]-[0070], examples<br>TW 202313540 A<br>KR 10-2389695 B1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020132624 A
- JP 2021520401 A
- WO 2023277347 A
- JP 2001010999 A
- JP 2005279587 A
- JP 2023148245 A
- JP 2024017280 A


### Non-patent literature cited in the description


- *Hitachi Chemical technical report* (51), 7-12
- *Applied Catalyst*, 1985, vol. 19, 259-273